# EUROPEAN PATENT APPLICATION

(11) **EP 4 644 385 A1**
(43) Date of publication of application: **05.11.2025**
(21) Application number: 25173293.9
(22) Date of filing: 29.04.2025
(51) Int. Cl.: C07D 401/14, A61P 35/00, A61K 31/506

(54) **TIPARP INHIBITOR COMPOUNDS**

(30) Priority: 30.04.2024 US 202463640741 P
(71) Applicant: AbbVie Inc., North Chicago, IL 60064 (US)
(72) Inventor: Bai, Wen Ju, Vernon Hills, 60061 (US); Cohen, Daniel, Wilmette, 60091 (US); Dai, Yujia, Gurnee, 60031 (US); De La Rosa, Martha A., Gurnee, 60031 (US); Dubovyk, Igor, San Jose, 95126 (US); Frey, Robin, Libertyville, 60048 (US); Ji, Zhiqin, Libertyville, 60048 (US); Judd, Andrew, Grayslake, 60030 (US); Liu, Dachun, Vernon Hills, 60061 (US); Mandal, Debashis, Hayward, 94544 (US); Punna, Sreenivas, Sunnyvale, 94085 (US)
(74) Representative: J A Kemp LLP

(57) **Abstract**

The present disclosure provides for compounds of Formula (I), and pharmaceutically acceptable salts thereof, that inhibit the activity of TIPARP, wherein the variables have any of the values defined in the specification.

## Description

### FIELD

The present disclosure pertains to compounds that inhibit the activity of 2,3,7,8-tetrachlorodibenzo-p-dioxin (TCDD)-inducible poly[adenosine diphosphate (ADP)-ribose] polymerase (TIPARP).

### BACKGROUND

The poly(ADP-ribose) polymerase (PARP) family of enzymes regulates fundamental cellular processes, including transcription, metabolism, and multiple cellular stress responses, through ADP-ribosylation. TIPARP, also known as PARP7, is a PARP enzyme that negatively regulates type I interferon (IFN) signaling. The gene responsible for encoding TIPARP is located in a region on chromosome 3q that frequently has copy number gains in some tumors, resulting in increased expression and restriction of anti-tumor immune responses. It is hypothesized that the inhibition of TIPARP in such tumors can restore type I IFN signaling and selectively activate anti-tumor immune responses in the tumor microenvironment, avoiding systematic cytokine production. (Joseph M. Gozgit, et al., PARP7 Negatively Regulates the Type I Interferon Response in Cancer Cells and Its Inhibition Triggers Antitumor Immunity, 39 CANCER CELL 1214 (2021)). However, off-target activity may prevent full engagement of the immune-mediated mechanism of action by some compounds that inhibit TIPARP. Compounds that lack sufficient selectivity for TIPARP may functionally inhibit other PARP enzymes (e.g., PARP1) at concentrations relevant for the inhibition of TIPARP, resulting in application-limiting side effects.

There remains a need in the art for improved compounds that selectively inhibit TIPARP. In particular, there remains a need in the art for compounds that exhibit high potency and high selectivity for TIPARP inhibition, while also exhibiting low inhibition of other PARP enzymes.

### SUMMARY

In one aspect, the invention provides for a compound of Formula (I), or a pharmaceutically acceptable salt thereof, 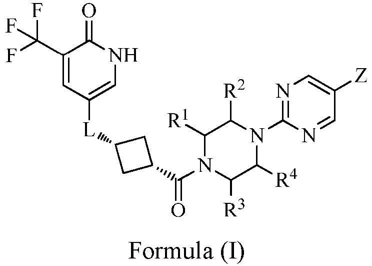 wherein:
L is selected from the group consisting of a bond and CH₂;
R¹, R², R³, and R⁴ are independently selected from the group consisting of H and CH₃, wherein at least one and no more than two of R¹, R², R³, and R⁴ is CH₃; and
Z is selected from the group consisting of CF₃ and C(CH₃)₂OH.

Another aspect of the invention provides for the compound of the first aspect, or a pharmaceutically acceptable salt thereof, wherein L is a bond.

Another aspect of the invention provides for the compound of the second aspect, or a pharmaceutically acceptable salt thereof, wherein one of R¹ or R⁴ is CH₃; both R¹ and R⁴ are CH₃; one of R² or R³ is CH₃; or both R² and R³ are CH₃.

Another aspect of the invention provides for the compound of the second or third aspect, or a pharmaceutically acceptable salt thereof, wherein Z is CF₃.

Another aspect of the invention provides for the compound of the first aspect, or a pharmaceutically acceptable salt thereof, wherein L is CH₂.

Another aspect of the invention provides for the compound of the fifth aspect, or a pharmaceutically acceptable salt thereof, wherein one of R¹ or R⁴ is CH₃; both R¹ and R⁴ are CH₃; one of R² or R³ is CH₃; or both R² and R³ are CH₃.

Another aspect of the invention provides for the compound of the fifth or sixth aspect, or a pharmaceutically acceptable salt thereof, wherein Z is C(CH₃)₂OH.

Another aspect of the invention provides for the compound of the first aspect, wherein the compound is selected from the group consisting of:
5-[(1*S*,3*s*)-3-{(2*R*)-2-methyl-4-[5-(trifluoromethyl)pyrimidin-2-yl]piperazine-1-carbonyl}cyclobutyl]-3-(trifluoromethyl)pyridin-2(1*H*)-one;
5-[(1*s*,3*s*)-3-{(2*R*,6*S*)-2,6-dimethyl-4-[5-(trifluoromethyl)pyrimidin-2-yl]piperazine-1-carbonyl}cyclobutyl]-3-(trifluoromethyl)pyridin-2(1*H*)-one;
5-[(1*S*,3*s*)-3-{(3*R*)-3-methyl-4-[5-(trifluoromethyl)pyrimidin-2-yl]piperazine-1-carbonyl}cyclobutyl]-3-(trifluoromethyl)pyridin-2(1*H*)-one;
5-[(1*s*,3*s*)-3-{(3*R*,5*S*)-3,5-dimethyl-4-[5-(trifluoromethyl)pyrimidin-2-ylpiperazine-1-carbonyl}cyclobutyl]-3-(trifluoromethyl)pyridin-2(1*H*)-one;
5-[(1*s*,3*s*)-3-{(3*R*,5*S*)-4-[5-(2-hydroxypropan-2-yl)pyrimidin-2-yl]-3,5-dimethylpiperazine-1-carbonyl}cyclobutyl]-3-(trifluoromethyl)pyridin-2(1*H*)-one;
5-[(1*R*,3*s*)-3-{(2*S*)-2-methyl-4-[5-(trifluoromethyl)pyrimidin-2-yl]piperazine-1-carbonyl}cyclobutyl]-3-(trifluoromethyl)pyridin-2(1*H*)-one;
5-[(1*S*,3*s*)-3-{(3*R*)-4-[5-(2-hydroxypropan-2-yl)pyrimidin-2-yl]-3-methylpiperazine-1-carbonyl}cyclobutyl]-3-(trifluoromethyl)pyridin-2(1*H*)-one;
5-[(1*R*,3*s*)-3-{(3*S*)-3-methyl-4-[5-(trifluoromethyl)pyrimidin-2-yl]piperazine-1-carbonyl}cyclobutyl]-3-(trifluoromethyl)pyridin-2(1*H*)-one;
5-[(1*S*,3*s*)-3-{(2*R*)-4-[5-(2-hydroxypropan-2-yl)pyrimidin-2-yl]-2-methylpiperazine-1-carbonyl}cyclobutyl]-3-(trifluoromethyl)pyridin-2(1*H*)-one;
5-[(1*R*,3*s*)-3-{(3*S*)-4-[5-(2-hydroxypropan-2-yl)pyrimidin-2-yl]-3-methylpiperazine-1-carbonyl}cyclobutyl]-3-(trifluoromethyl)pyridin-2(1*H*)-one;
5-[(1*R*,3*s*)-3-{(2*S*)-4-[5-(2-hydroxypropan-2-yl)pyrimidin-2-yl]-2-methylpiperazine-1-carbonyl}cyclobutyl]-3-(trifluoromethyl)pyridin-2(1*H*)-one;
5-{[(1*S*,3*s*)-3-{(2*R*)-2-methyl-4-[5-(trifluoromethyl)pyrimidin-2-yl]piperazine-1-carbonyl}cyclobutyl]methyl}-3-(trifluoromethyl)pyridin-2(1*H*)-one;
5-{[(1*s*,3*s*)-3-{(2*R*,6*S*)-2,6-dimethyl-4-[5-(trifluoromethyl)pyrimidin-2-yl]piperazine-1-carbonyl}cyclobutyl]methyl}-3-(trifluoromethyl)pyridin-2(1*H*)-one;
5-{[(1*s*,3*s*)-3-{(3*R*,5*S*)-3,5-dimethyl-4-[5-(trifluoromethyl)pyrimidin-2-yl]piperazine-1-carbonyl}cyclobutyl]methyl}-3-(trifluoromethyl)pyridin-2(1*H*)-one;
5-{[(1*S*,3*s*)-3-{(3*R*)-3-methyl-4-[5-(trifluoromethyl)pyrimidin-2-yl]piperazine-1-carbonyl}cyclobutyl]methyl}-3-(trifluoromethyl)pyridin-2(1*H*)-one;
5-{[(1*R*,3*s*)-3-{(3*S*)-3-methyl-4-[5-(trifluoromethyl)pyrimidin-2-yl]piperazine-1-carbonyl}cyclobutyl]methyl}-3-(trifluoromethyl)pyridin-2(1*H*)-one;
5-{[(1*R*,3*s*)-3-{(2*S*)-2-methyl-4-[5-(trifluoromethyl)pyrimidin-2-yl]piperazine-1-carbonyl}cyclobutyl]methyl}-3-(trifluoromethyl)pyridin-2(1*H*)-one;
5-{[(1*S*,3*s*)-3-{(3*R*)-4-[5-(2-hydroxypropan-2-yl)pyrimidin-2-yl]-3-methylpiperazine-1-carbonyl}cyclobutyl]methyl}-3-(trifluoromethyl)pyridin-2(1*H*)-one;
5-{[(1*S*,3*s*)-3-{(2*R*)-4-[5-(2-hydroxypropan-2-yl)pyrimidin-2-yl]-2-methylpiperazine-1-carbonyl}cyclobutylmethyl}-3-(trifluoromethyl)pyridin-2(1*H*)-one;
5-{[(1*R*,3*s*)-3-{(3*S*)-4-[5-(2-hydroxypropan-2-yl)pyrimidin-2-yl]-3-methylpiperazine-1-carbonyl}cyclobutyl]methyl}-3-(trifluoromethyl)pyridin-2(1*H*)-one;
5-{[(1*R*,3*s*)-3-{(2*S*)-4-[5-(2-hydroxypropan-2-yl)pyrimidin-2-yl]-2-methylpiperazine-1-carbonyl}cyclobutyl]methyl}-3-(trifluoromethyl)pyridin-2(1*H*)-one; and
5-{[(1*s*,3*s*)-3-{(3*R*,5*S*)-4-[5-(2-hydroxypropan-2-yl)pyrimidin-2-yl]-3,5-dimethylpiperazine-1-carbonyl}cyclobutyl]methyl}-3-(trifluoromethyl)pyridin-2(1*H*)-one.

Another aspect of the invention provides for 5-[(1S,3s)-3-{(2R)-2-methyl-4-[5-(trifluoromethyl)pyrimidin-2-yl]piperazine-1-carbonyl}cyclobutyl]-3-(trifluoromethyl)pyridin-2(1*H*)-one, or a pharmaceutically acceptable salt thereof.

Another aspect of the invention provides for 5-[(1*S*,3*s*)-3-{(2*R*)-2-methyl-4-[5-(trifluoromethyl)pyrimidin-2-yl]piperazine-1-carbonyl}cyclobutyl]-3-(trifluoromethyl)pyridin-2(1*H*)-one.

Another aspect of the invention provides for a pharmaceutically acceptable salt of 5-[(1*S*,3*s*)-3-{(2*R*)-2-methyl-4-[5-(trifluoromethyl)pyrimidin-2-yl]piperazine-1-carbonyl}cyclobutyl]-3-(trifluoromethyl)pyridin-2(1*H*)-one.

Another aspect of the invention provides for a pharmaceutical composition comprising the compound of any of the aspects above, or a pharmaceutically acceptable salt thereof, and a pharmaceutically acceptable excipient.

Another aspect of the invention provides for a method for treating head and neck squamous cell carcinoma (HNSCC), the method comprising administering the compound of the first aspect, or a pharmaceutically acceptable salt thereof, to a human patient in need thereof. The invention also provides a compound of any of the aspects above, or a pharmaceutically acceptable salt thereof, for use in a method for treating head and neck squamous cell carcinoma (HNSCC). The invention also provides a pharmaceutical composition comprising the compound of any of the aspects above, or a pharmaceutically acceptable salt thereof, for use in a method for treating head and neck squamous cell carcinoma.

### BRIEF DESCRIPTION OF THE DRAWINGS

FIG. 1 depicts the *in vivo* efficacy of Example 1 in reducing tumor volume in the MC-38 (Ker) syngeneic model.
FIG. 2 depicts the induction of IFNβ in plasma and tumors in the MC-38 (Ker) syngeneic model after the administration of Example 1.
FIG. 3 depicts the *in vivo* efficacy of Example 1 in reducing tumor volume in an NCI-H1373 xenograft model.

### DETAILED DESCRIPTION

The present invention provides for compounds that inhibit the activity of TIPARP.

Compounds disclosed herein, including any intermediates, may contain one or more variable(s) that occur more than one time in any substituent or in the Formulae herein. Definition of a variable on each occurrence is independent of its definition at another occurrence.

Compounds disclosed herein, including any intermediates, were named by using ACD/Name 2023.1.2 (File Version N25E41, Build 134315, 12 July 2023) software program and/or by using Struct=Name naming algorithm as part of CHEMDRAW^{®} Professional v. 20.1.1.125.

Compounds disclosed herein, including any intermediates, may possess multiple tautomeric forms and exist as equilibrium mixtures thereof. The formulae and structures found herein represent only one of the possible tautomeric forms but should be understood to encompass both individual tautomeric forms and mixtures thereof.

### DEFINITIONS

As used in the specification and the appended claims, unless specified to the contrary, the following terms have the meaning indicated:

The phrase "pharmaceutical composition" refers to a composition suitable for administration in medical use.

The phrase "pharmaceutically acceptable salt" refers to those salts which are, within the scope of sound medical judgement, suitable for use in contact with the tissues of humans and lower animals without undue toxicity, irritation, allergic response and the like and are commensurate with a reasonable benefit/risk ratio.

The phrase "therapeutically effective amount" refers to an amount of a compound, or a pharmaceutically acceptable salt thereof, sufficient to prevent the development of or to alleviate to some extent one or more of the symptoms of the condition or disorder being treated when administered for treatment in a particular human patient or human patient population.

The terms "treat," "treating," and "treatment," as used herein, refer to a method of alleviating or abrogating a disease and/or its attendant symptoms.

### COMPOUNDS

The present disclosure provides for compounds of Formula (I), or pharmaceutically acceptable salts thereof, 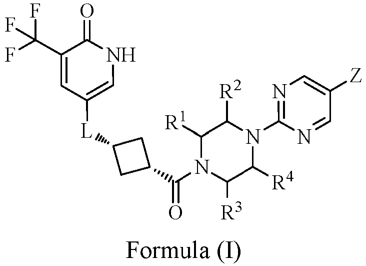 wherein:
L is selected from the group consisting of a bond and CH₂;
R¹, R², R³, and R⁴ are independently selected from the group consisting of H and CH₃, wherein at least one and no more than two of R¹, R², R³, and R⁴ is CH₃; and
Z is selected from the group consisting of CF₃ and C(CH₃)₂OH.

Exemplary compounds of Formula (I) are shown in Table 1 below. It is to be understood that when there is a discrepancy between the name of any compounds disclosed herein and the structures found in Table 1, the structures in Table 1 shall prevail.

**Table 1**

| **Ex. #** | **Structure** | **Ex. #** | **Structure** |
|---|---|---|---|
| **1** | 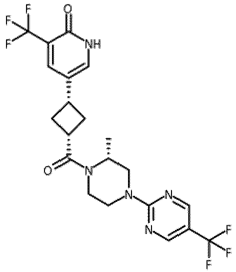 | **12** | 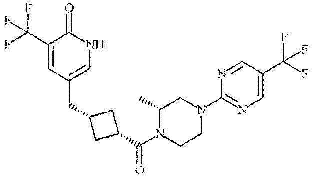 |
| **2** | 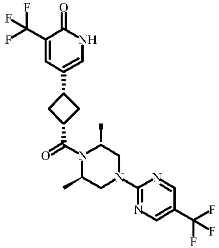 | **13** | 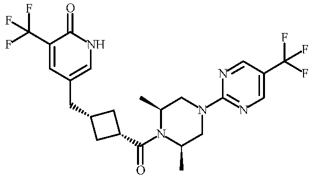 |
| **3** | 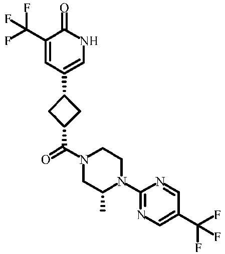 | **14** | 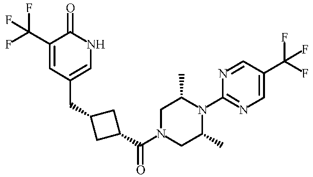 |
| **4** | 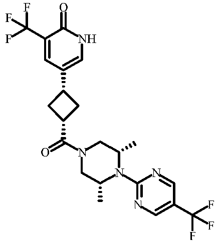 | **15** | 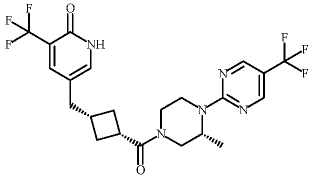 |
| **5** | 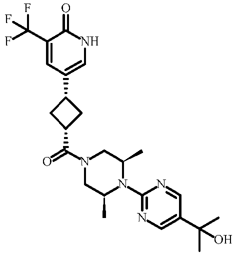 | **16** | 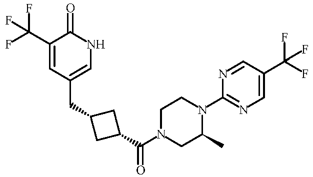 |
| **6** | 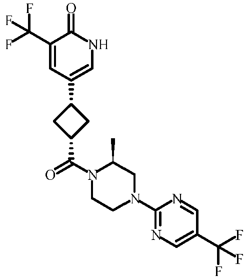 | **17** | 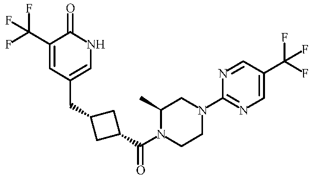 |
| **7** | 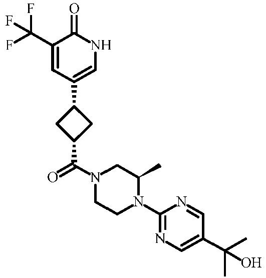 | **18** | 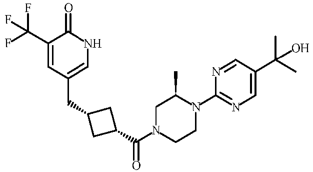 |
| **8** | 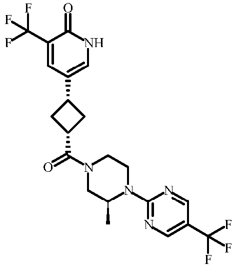 | **19** | 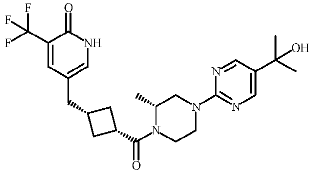 |
| **9** | 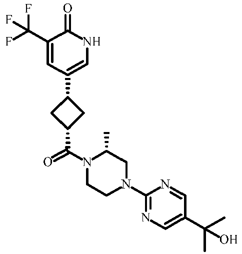 | **20** | 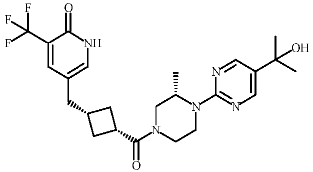 |
| **10** | 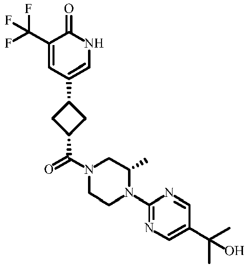 | **21** | 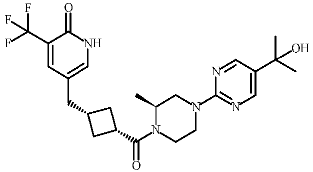 |
| **11** | 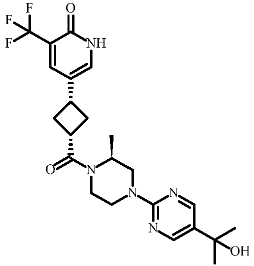 | **22** | 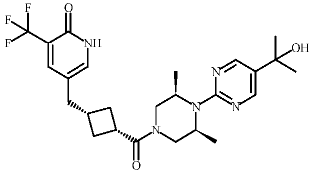 |

Compounds of Formula (I) may be used in the form of pharmaceutically acceptable salts. Such compounds may contain either a basic or an acidic functionality, or both, and may be converted to a pharmaceutically acceptable salt, when desired, by using a suitable acid or base.

The compounds of Formula (I) exhibit a combination of functional properties, including but not limited to, high potency for inhibiting TIPARP, high selectivity for inhibiting TIPARP compared to other PARP family enzymes, and low inhibition of PARP1 and/or PARP2. The compounds of Formula (I) may exhibit high potency and selectivity for inhibiting TIPARP. Additionally, the compounds of Formula (I) may exhibit low inhibition of PARP enzymes other than TIPARP. As used herein, low inhibition of PARP enzymes other than TIPARP means the compounds of Formula (I) lack sufficient potency for inhibiting PARP enzymes other than TIPARP, For example, the compounds of Formula (I) may exhibit low inhibition of PARP1, PARP2, or combinations thereof.

### METHODS OF MAKING EXEMPLARY COMPOUNDS

The compounds of the present disclosure may be better understood in connection with the following synthetic schemes and methods which illustrate a means by which the compounds can be prepared. The compounds of the present disclosure can be prepared by a variety of synthetic procedures. Representative synthetic procedures are shown in **Schemes 1-8.** The variables have any of the values defined herein, e.g., in the Summary.

### SYNTHETIC SCHEMES

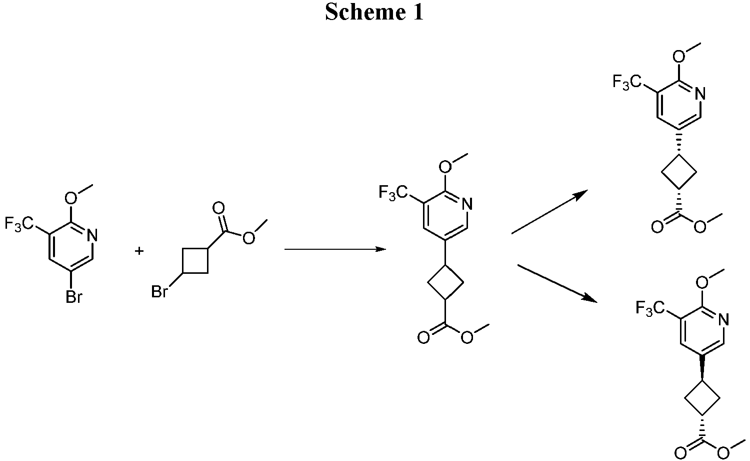

As shown in **Scheme 1,** methyl 3-(6-methoxy-5-(trifluoromethyl)pyridin-3-yl)cyclobutanecarboxylate can be prepared by reacting 5-bromo-2-methoxy-3-(trifluoromethyl)pyridine with methyl 3-bromocyclobutanecarboxylate. The reaction may be performed in the presence of a cross coupling catalyst, such as (4,4'-dtbbpy)NiCl₂, a photocatalyst, such as (Ir[dF(CF₃)ppy]₂(dtbpy))PF₆, 1,1,1,3,3,3-hexamethyl-2-(trimethylsilyl)trisilane, 2,6-lutidine, and LED blue light. The reaction may be performed in an inert atmosphere in a solvent, such as 1,2-dimethoxyethane. The trans-isomer may be separated from the cis-isomer via flash chromatography.

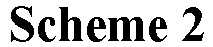

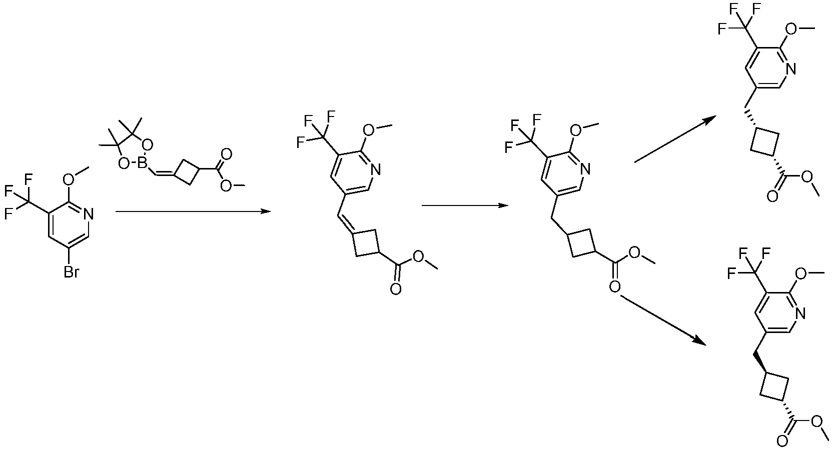

As shown in **Scheme 2,** 5-bromo-2-methoxy-3-(trifluoromethyl)pyridine can be reacted under Suzuki coupling conditions with methyl 3-((4,4,5,5-tetramethyl-1,3,2-dioxaborolan-2-yl)methylene)cyclobutane-1-carboxylate, in the presence of a catalyst, such as Pd(dppf)Cl₂·CH₂Cl₂, and a base, such as potassium carbonate, to provide methyl 3-((6-methoxy-5-(trifluoromethyl)pyridin-3-yl)methylene)cyclobutane-1-carboxylate. The reaction is typically performed in an inert atmosphere at an elevated temperature and in a solvent, such as dioxane, water, or mixtures thereof.

Methyl 3-((6-methoxy-5-(trifluoromethyl)pyridin-3-yl)methylene)cyclobutane-1-carboxylate can be treated with hydrogen gas in the presence of a catalyst, such as Pd/C, to provide methyl 3-((6-methoxy-5-(trifluoromethyl)pyridin-3-yl)methyl)cyclobutane-1-carboxylate, which may contain a mixture of separable cis- and trans-isomers. The trans-isomer may be separated from the cis-isomer via flash chromatography. The reaction is typically performed at ambient temperature in a solvent, such as tetrahydrofuran.

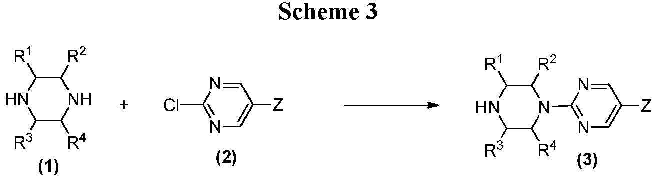

**Scheme 3** shows the synthesis of compounds of Formula (3), wherein R¹, R², R³, R⁴ and Z are as described herein. Compounds of Formula (1) can be reacted with compounds of Formula (2) in the presence of a base, such as triethylamine, to provide compounds of Formula (3). The reaction is typically performed in a solvent, such as acetonitrile.

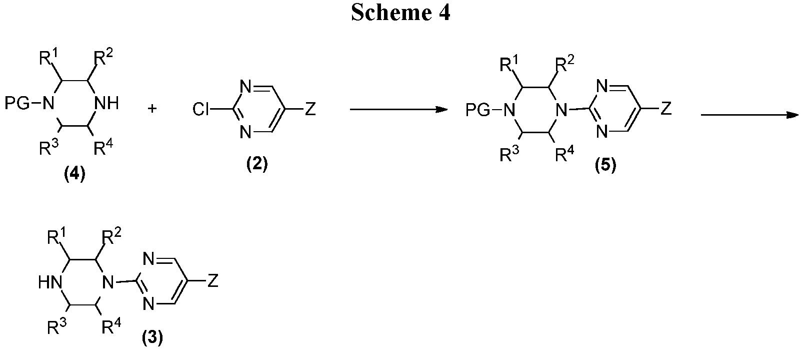

**Scheme 4** shows an alternate synthesis of compounds of Formula (3), wherein R¹, R², R³, R⁴, and Z are as described herein. Compounds of Formula (4), wherein PG is a protecting group such as a BOC group, can be reacted with compounds of Formula (2) in the presence of a base, such as triethylamine or *N*,*N-*diisopropylethylamine, to provide compounds of Formula (5). The reaction is typically performed in a solvent such as acetonitrile or dimethylacetamide. When PG is a BOC group, compounds of Formula (5) can be treated with an acid, such as trifluoroacetic acid or hydrogen chloride, in a solvent, such as dichloromethane, acetonitrile, dioxane, or mixtures thereof, to provide compounds of Formula (3).

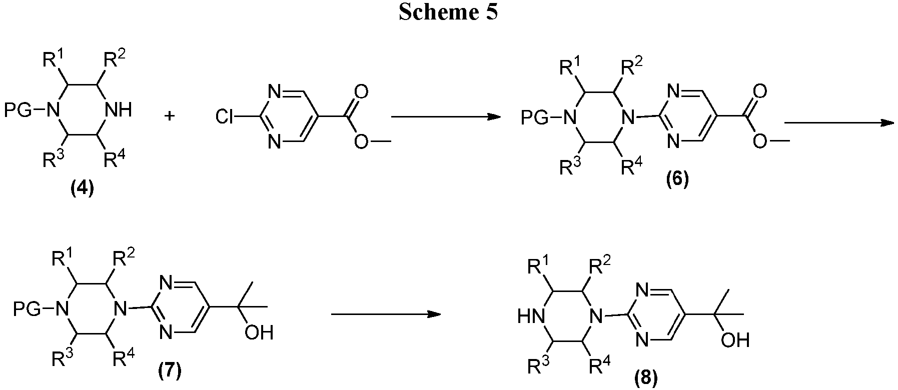

**Scheme 5** shows the synthesis of compounds of Formula (8), wherein R¹, R², R³, and R⁴ are as described herein. Compounds of Formula (4) can be reacted with methyl 2-chloropyrimidine-5-carboxylate in the presence of a base, such as potassium carbonate, to provide compounds of Formula (6). The reaction is typically performed at an elevated temperature in a solvent, such as *N*,*N-*dimethylformamide.

Alternatively, compounds of Formula (4) can be reacted with methyl 2-chloropyrimidine-5-carboxylate in *N*-methyl-2-pyrrolidinone to provide compounds of Formula (6). The reaction is typically performed at an elevated temperature.

Compounds of Formula (6) can be treated with methylmagnesium bromide solution to provide compounds of Formula (7). The reaction is typically performed under nitrogen at low temperature in a solvent, such as tetrahydrofuran. When PG is a BOC group, compounds of Formula (7) can be treated with an acid, such as trifluoroacetic acid or hydrogen chloride, in a solvent, such as dichloromethane, ethyl acetate, acetonitrile, dioxane, or mixtures thereof, to provide compounds of Formula (8).

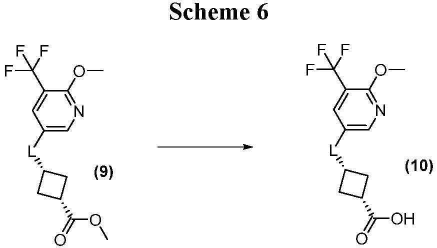

As shown in **Scheme 6,** the treatment of compounds of Formula (9), which can be prepared as described in **Schemes 1-2,** with lithium hydroxide or sodium hydroxide in water can provide compounds of Formula (10). The reaction is typically performed at ambient temperature in a solvent, such as methanol, tetrahydrofuran, or mixtures thereof.

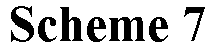

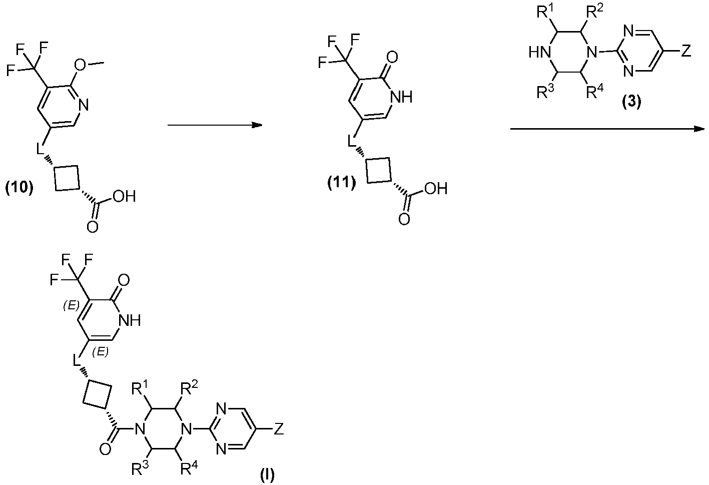

As shown in **Scheme 7,** compounds of Formula (10) can be treated with *para-*toluenesulfonic acid monohydrate and lithium chloride to provide compounds of Formula (11). The reaction is typically performed at an elevated temperature in a solvent, such as *N,N-*dimethylformamide.

Compounds of Formula (I) can be prepared by reacting compounds of Formula (11), wherein L is a bond or CH₂, with compounds of Formula (3) in the presence of propanephosphonic acid anhydride and a base, such as *N*,*N-*diisopropylethylamine. The reaction is typically performed at ambient temperature in a solvent such as *N*,*N-*dimethylformamide.

Alternatively, when L is a bond or CH₂, compounds of Formula (I) can be prepared by reacting compounds of Formula (11) with compounds of Formula (3) in the presence of 1-[bis(dimethylamino)methylene]-1*H*-1,2,3-triazolo[4,5-*b*]pyridinium 3-oxid hexafluorophosphate and a base, such as *N*,*N-*diisopropylethylamine. The reaction is typically performed at ambient or elevated temperatures in a solvent such as *N,N-*dimethylformamide.

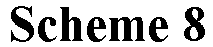

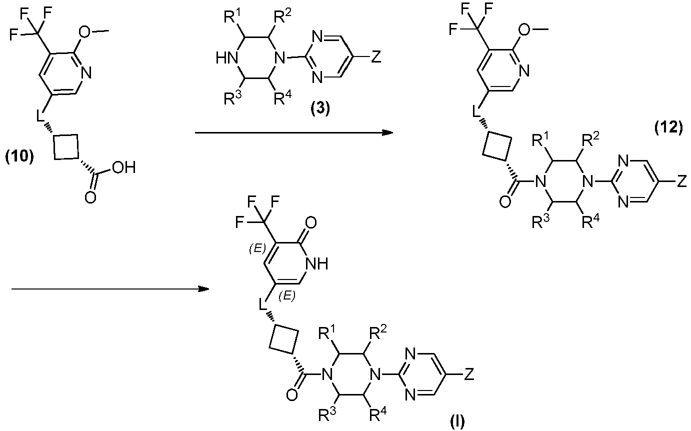

As shown in **Scheme 8,** compounds of Formula (12) can be prepared by reacting compounds of Formula (10), wherein L is a bond or CH₂, with compounds of Formula (3) in the presence of propanephosphonic acid anhydride and a base, such as *N*,*N-*diisopropylethylamine. The reaction is typically performed at ambient temperature in a solvent such as *N,N-*dimethylformamide.

Compounds of Formula (12) can be treated with para-toluenesulfonic acid monohydrate and lithium chloride to provide compounds of Formula (I). The reaction is typically performed in an inert atmosphere at an elevated temperature in a solvent such as *N*,*N-*dimethylformamide.

Specific procedures are also provided in the Synthetic Examples section. Unless otherwise described, the starting materials and reagents are either commercially available or may be prepared by one skilled in the art from commercially available materials using methods known in the art.

### PHARMACEUTICAL COMPOSITIONS

When employed as a pharmaceutical, a compound of the present disclosure may be administered in the form of a pharmaceutical composition. Such composition may comprise a therapeutically effective amount of a compound of Formula (I), or a pharmaceutically acceptable salt thereof, together with one or more pharmaceutically acceptable excipients.

### METHODS OF USE

The compounds of Formula (I), or pharmaceutically acceptable salts thereof, and pharmaceutical compositions comprising a compound of Formula (I), or a pharmaceutically acceptable salt thereof, may be administered to a human patient suffering from head and neck squamous cell carcinoma (HNSCC). The term "administering" refers to the method of contacting a human patient with a compound.

The compounds of Formula (I), or pharmaceutically acceptable salts thereof, may also be used in the preparation of a medicament. Such medicament may be used in the treatment of HNSCC.

### EXAMPLES

### SYNTHETIC EXAMPLES

### Example 1

### 5-[(1S,3s)-3-{(2R)-2-methyl-4-[5-(trifluoromethyl)pyrimidin-2-yl]piperazine-1-carbonyl}cyclobutyl]-3-(trifluoromethyl)pyridin-2(1H)-one

### Example 1A

### (1s,3s)-methyl 3-(6-methoxy-5-(trifluoromethyl)pyridin-3-yl)cyclobutanecarboxylate

In Example 1A, 5-Bromo-2-methoxy-3-(trifluoromethyl)pyridine (5.12 g, 20.0 mmol), methyl 3-bromocyclobutanecarboxylate (4.63 g, 24.0 mmol), (4,4'-dtbbpy)NiCl₂ (398 mg, 1.00 mmol, CAS 1034901-50-2), (Ir[dF(CF₃)ppy]₂(dtbpy))PF₆ (224 mg, 0.200 mmol, CAS 870987-63-6), 1,1,1,3,3,3-hexamethyl-2-(trimethylsilyl)trisilane (5.97 g, 24.0 mmol) and 2,6-lutidine (5.82 mL, 50.0 mmol) were combined with 1,2-dimethoxyethane (80 mL). The reaction mixture was purged with nitrogen for 5 minutes and stirred for 16 hours under irradiation (450 nm LED blue light). The reaction mixture was partitioned between ethyl acetate and water. The organic layer was washed with saturated aqueous sodium chloride, dried with anhydrous sodium sulfate, filtered, and concentrated under vacuum. The residue was purified by flash chromatography (silica gel, 0-30% ethyl acetate in heptanes) to give two fractions. The first eluted fraction was the stereoisomer with trans-configuration. The second eluted fraction was the title compound with cis-configuration. MS (ESI) *m*/*z* 290 (M+H)⁺.

### Example 1B

### (1s,3s)-3-(6-methoxy-5-(trifluoromethyl)pyridin-3-yl)cyclobutanecarboxylic acid

To a solution of Example 1A (900 mg, 3.11 mmol) in the mixture of tetrahydrofuran (6.0 mL)/methanol (6.0 mL)/water (2.0 mL) was added sodium hydroxide (622 mg, 15.6 mmol). The reaction mixture was stirred at room temperature for 1 hour and diluted with water. The pH was adjusted to 4 with the addition of 1M aqueous HCl and extracted with ethyl acetate. The organic layer was washed with saturated aqueous sodium chloride, dried with anhydrous sodium sulfate, and filtered. The filtrate was concentrated under vacuum to provide the title compound. MS (ESI) *m*/*z* 276 (M+H)⁺.

### Example 1C

### (1s,3s)-3-(6-oxo-5-(trifluoromethyl)-1,6-dihydropyridin-3-yl)cyclobutanecarboxylic acid

Example 1B (730 mg, 2.65 mmol), *para*-toluenesulfonic acid monohydrate (1.01 g, 5.30 mmol), and lithium chloride (562 mg, 13.3 mmol) were combined in *N*,*N-*dimethylformamide (15 mL). The reaction mixture was stirred at 100 °C for 10 hours, cooled to room temperature, and partitioned between ethyl acetate and water. The organic layer was washed with saturated aqueous sodium chloride, dried with anhydrous sodium sulfate, and filtered. The filtrate was concentrated under vacuum to provide the title compound. MS (ESI) *m*/*z* 262 (M+H)⁺.

### Example 1D

### (R)-2-(3-methylpiperazin-1-yl)-5-(trifluoromethyl)pyrimidine

In Example 1D, a solution of (*R*)-2-methylpiperazine (2.50 g, 25.0 mmol) and triethylamine (6.96 mL, 49.9 mmol) in acetonitrile (80 mL) was cooled to 0 °C in an ice bath, and treated with 2-chloro-5-(trifluoromethyl)pyrimidine (4.56 g, 25.0 mmol). The reaction mixture was stirred at room temperature overnight. The insoluble salt material was filtered and washed with a small amount of acetonitrile. The filtrate was concentrated under vacuum. The corresponding residue was triturated with diethyl ether (25 mL), filtered, and dried under vacuum to provide the title compound. The filtrate was concentrated under vacuum to about 8 mL, placed in a refrigerator overnight, and the mixture was filtered. The material was dried under vacuum to provide additional title compound. MS (APCI) *m*/*z* 288 (M+CH₃CN+H)⁺.

### Example 1E

### 5-[(1S,3s)-3-{(2R)-2-methyl-4-[5-(trifluoromethyl)pyrimidin-2-yl]piperazine-1-carbonyl}cyclobutyl]-3-(trifluoromethyl)pyridin-2(1H)-one

To a mixture of Example 1C (20.9 mg, 0.0800 mmol), Example 1D (22.6 mg, 0.0920 mmol) and *N,N*-diisopropylethylamine (0.0840 mL, 0.480 mmol) in *N,N-*dimethylformamide (1.0 mL) was added 50% propanephosphonic acid anhydride in *N,N-*dimethylformamide (102 mg, 0.160 mmol). The reaction mixture was stirred at room temperature for 1 hour and partitioned between ethyl acetate and water. The organic layer was washed with saturated aqueous sodium chloride, dried with anhydrous sodium sulfate, filtered, concentrated, and purified by flash chromatography to provide the title compound. ¹H NMR (400 MHz, DMSO-*d*₆, 90 °C) *δ* ppm 11.84 (s, br, 1H), 8.64 (d, J = 0.8 Hz, 2H), 7.77 (d, J = 2.6 Hz, 1H), 7.42 (d, J = 2.6 Hz, 1H), 4.64 - 4.29 (m, 3H), 3.96 (s, br, 1H), 3.38 - 3.24 (m, 3H), 3.23 - 3.04 (m, 2H), 2.54 - 2.45 (m, 2H), 2.25 - 2.09 (m, 2H), 1.08 (d, J = 6.7 Hz, 3H). MS (ESI) *m*/*z* 490 (M+H)⁺.

### Example 2

### 5-[(1s,3s)-3-{(2R,6S)-2,6-dimethyl-4-[5-(trifluoromethyl)pyrimidin-2-yl]piperazine-1-carbonyl}cyclobutyl]-3-(trifluoromethyl)pyridin-2(1H)-one

Example 2 was prepared according to the procedure used for the preparation of Example 13F, substituting Example 1C for Example 13D, to provide the title compound. ¹H NMR (400 MHz, DMSO-*d*₆, 90 °C) *δ* ppm 11.84 (s, br, 1H), 8.65 (d, J = 0.8 Hz, 2H), 7.79 (d, J = 2.6 Hz, 1H), 7.43 (d, J = 2.6 Hz, 1H), 4.60 - 4.52 (m, 2H), 4.41 (s, br, 2H), 3.37 - 3.20 (m, 4H), 2.54 - 2.45 (m, 2H), 2.25 - 2.16 (m, 2H), 1.17 (d, J = 7.0 Hz, 6H). MS (ESI) *m*/*z* 504 (M+H)⁺.

### Example 3

### 5-[(1S,3s)-3-{(3R)-3-methyl-4-[5-(trifluoromethyl)pyrimidin-2-yl]piperazine-1-carbonyl}cyclobutyl]-3-(trifluoromethyl)pyridin-2(1H)-one

### Example 3A

### (R)-tert-butyl 3-methyl-4-(5-(trifluoromethyl)pyrimidin-2-yl)piperazine-1-carboxylate

In Example 3A, a mixture of 2-chloro-5-(trifluoromethyl)pyrimidine (5.00 g, 27.4 mmol), (*R*)-*tert*-butyl 3-methylpiperazine-1-carboxylate (5.49 g, 27.4 mmol) and triethylamine (7.64 mL, 54.8 mmol) in acetonitrile (100 mL) was stirred at 80 °C for 3 hours, and cooled to room temperature. The insoluble salt material was filtered and washed with a small amount of acetonitrile. The filtrate was concentrated under vacuum. To the residue was added water, and the mixture was extracted with ethyl acetate twice. The combined organic layers were washed with saturated aqueous sodium chloride, dried with anhydrous magnesium sulfate, and filtered. The filtrate was concentrated under vacuum. The residue was purified by flash chromatography (silica gel, 0-25% ethyl acetate in heptanes) to provide the title compound. MS (APCI) *m*/*z* 347 (M+H)⁺.

### Example 3B

### (R)-2-(2-methylpiperazin-1-yl)-5-(trifluoromethyl)pyrimidine

A solution of Example 3A (8.02 g, 23.2 mmol) in dioxane (50 mL) was treated with 4M hydrogen chloride in dioxane (57.9 ml, 232 mmol) at 0 °C. The reaction mixture was stirred at room temperature overnight and filtered. The filter cake was washed with diethyl ether and dried under vacuum to provide the title compound as a hydrochloric acid salt. MS (ESI) *m*/*z* 247 (M+H)⁺.

### Example 3C

### 5-[(1S,3s)-3-{(3R)-3-methyl-4-[5-(trifluoromethyl)pyrimidin-2-yl]piperazine-1-carbonyl}cyclobutyl]-3-(trifluoromethyl)pyridin-2(1H)-one

To a mixture of Example 1C (20 mg, 0.077 mmol), Example 3B (22 mg, 0.077 mmol) and *N,N*-diisopropylethylamine (0.080 mL, 0.46 mmol) in *N*,*N-*dimethylformamide (1.0 mL) was added 50% propanephosphonic acid anhydride in *N*,*N-*dimethylformamide (97 mg, 0.15 mmol). The reaction mixture was stirred at room temperature for 1 hour, and purified by reverse phase HPLC (C18, 20-100% acetonitrile in water with 0.1% trifluoroacetic acid). The acetonitrile was removed by evaporation, and the mixture was neutralized with saturated aqueous sodium bicarbonate. The mixture was extracted with ethyl acetate and the organic layer was dried with anhydrous sodium sulfate and filtered. The filtrate was concentrated under vacuum to provide the title compound. ¹H NMR (400 MHz, DMSO-*d*₆, 90 °C) *δ* ppm 11.84 (s, 1H), 8.66 (d, *J* = 0.8 Hz, 2H), 7.78 (d, *J =* 2.6 Hz, 1H), 7.43 (d, *J =* 2.6 Hz, 1H), 4.94 - 4.85 (m, 1H), 4.52 - 4.43 (m, 1H), 4.14 (s, br, 1H), 3.85 (s, br, 1H), 3.39 - 3.23 (m, 3H), 3.07 (s, br, 2H), 2.55 - 2.49 (m, 2H), 2.24 - 2.14 (m, 2H), 1.14 (d, *J* = 6.7 Hz, 3H). MS (ESI) *m*/*z* 490 (M+H)⁺.

### Example 4

### 5-[(1s,3s)-3-{(3R,5S)-3,5-dimethyl-4-[5-(trifluoromethyl)pyrimidin-2-yl]piperazine-1-carbonyl}cyclobutyl]-3-(trifluoromethyl)pyridin-2(1H)-one

### Example 4A

### (3R,5S)-tert-butyl 3,5-dimethyl-4-(5-(trifluoromethyl)pyrimidin-2-yl)piperazine-1-carboxylate

To a solution of *tert-butyl* (3*R*,5*S*)-3,5-dimethylpiperazine-1-carboxylate (2.00 g, 9.33 mmol) in dimethylacetamide (18.7 mL) was added *N*,*N*-diisopropylethylamine (2.45 ml, 14.0 mmol) and 2-chloro-5-(trifluoromethyl)pyrimidine (1.70 g, 9.33 mmol). The reaction mixture was stirred at 90 °C for 18 hours, cooled to room temperature, diluted with water, and extracted with ethyl acetate. The organic layer was washed with saturated aqueous sodium chloride twice, dried with anhydrous magnesium sulfate, and filtered. The filtrate was concentrated under vacuum. The residue was purified by flash chromatography (silica gel, 1-20% ethyl acetate in heptanes) to provide the title compound. MS (ESI) *m*/*z* 305 (M-56+H)⁺.

### Example 4B

### 2-((2R,6S)-2,6-dimethylpiperazin-1-yl)-5-(trifluoromethyl)pyrimidine

A solution of Example 4A (2.18 g, 6.05 mmol) in dioxane (20.2 mL) was treated with 4M hydrogen chloride in dioxane (15.1 ml, 60.4 mmol). The reaction mixture was stirred at room temperature overnight, filtered, and the filter cake was dried under vacuum to provide the title compound as a hydrochloric acid salt. MS (ESI) *m*/*z* 261 (M+H)⁺.

### Example 4C

### 5-[(1s,3s)-3-{(3R,5S)-3,5-dimethyl-4-[5-(trifluoromethyl)pyrimidin-2-yl]piperazine-1-carbonyl}cyclobutyl]-3-(trifluoromethyl)pyridin-2(1H)-one

Example 4C was prepared according to the procedure used for the preparation of Example 3C, substituting Example 4B for Example 3B, to provide the title compound. ¹H NMR (400 MHz, DMSO-*d*₆, 90 °C) *δ* ppm 11.82 (s, 1H), 8.68 (s, 2H), 7.80 (d, *J =* 2.6 Hz, 1H), 7.44 (d, *J* = 2.6 Hz, 1H), 4.89 - 4.79 (m, 2H), 4.05 (s, br, 2H), 3.45 - 3.24 (m, 2H), 3.11 (s, br, 2H), 2.57 - 2.50 (m, 2H), 2.29 - 2.16 (m, 2H), 1.19 (d, *J* = 6.9 Hz, 6H). MS (ESI) *m*/*z* 504 (M+H)⁺.

### Example 5

### 5-[(1s,3s)-3-{(3R,5S)-4-[5-(2-hydroxypropan-2-yl)pyrimidin-2-yl]-3,5-dimethylpiperazine-1-carbonyl}cyclobutyl]-3-(trifluoromethyl)pyridin-2(1H)-one

Example 5 was prepared according to the procedure used for the preparation of Example 1E, substituting Example 22C for Example 1D. Purification by flash chromatography provided the title compound. ¹H NMR (400 MHz, DMSO-*d*₆, 90 °C) *δ* ppm 11.84 (s, br, 1H), 8.44 (s, 2H), 7.79 (d, *J* = 2.6 Hz, 1H), 7.44 (d, *J* = 2.6 Hz, 1H), 4.84 - 4.67 (m, 3H), 3.97 (s, br, 2H), 3.48 - 3.22 (m, 4H), 2.56 - 2.49 (m, 2H), 2.27 - 2.18 (m, 2H), 1.43 (s, 6H), 1.14 (d, *J* = 6.8 Hz, 6H). MS (ESI) *m*/*z* 476 (M-H₂O+H)⁺.

### Example 6

### 5-[(1R,3s)-3-{(2S)-2-methyl-4-[5-(trifluoromethyl)pyrimidin-2-yl]piperazine-1-carbonyl}cyclobutyl]-3-(trifluoromethyl)pyridin-2(1H)-one

### Example 6A

### (S)-tert-butyl 2-methyl-4-(5-(trifluoromethyl)pyrimidin-2-yl)piperazine-1-carboxylate

In Example 6A, (*S*)-*tert*-Butyl 2-methylpiperazine-1-carboxylate (4.00 g, 20.0 mmol), 2-chloro-5-(trifluoromethyl)pyrimidine (3.65 g, 20.0 mmol) and triethylamine (5.57 mL, 39.9 mmol) were combined in acetonitrile (80 mL). The reaction mixture was stirred at room temperature overnight. The insoluble salt material was filtered. The filtrate was concentrated under vacuum. The residue was triturated with water, filtered, rinsed with water, and dried under vacuum to provide the title compound. MS (ESI) *m*/*z* 291 (M-56+H)⁺.

### Example 6B

### (S)-2-(3-methylpiperazin-1-yl)-5-(trifluoromethyl)pyrimidine

To a solution of Example 6A (6.20 g, 17.9 mmol) in acetonitrile (50 mL) was added 4M hydrogen chloride in dioxane (22.4 mL, 89.6 mmol). The reaction mixture was stirred at room temperature for 4 hours, diluted with *tert*-butyl methyl ether, and filtered. The material was dried under vacuum to provide the title compound as a hydrochloric acid salt. MS (ESI) *m*/*z 247* (M+H)⁺.

### Example 6C

### 5-[(1R,3s)-3-{(2S)-2-methyl-4-[5-(trifluoromethyl)pyrimidin-2-yl]piperazine-1-carbonyl}cyclobutyl]-3-(trifluoromethyl)pyridin-2(1H)-one

Example 6C was prepared according to the procedure used for the preparation of Example 1E, substituting Example 6B for Example 1D, to provide the title compound. ¹H NMR (400 MHz, DMSO-*d*₆, 90 °C) *δ* ppm 11.84 (s, br, 1H), 8.64 (d, *J* = 0.9 Hz, 2H), 7.77 (d, *J* = 2.6 Hz, 1H), 7.42 (d, *J =* 2.6 Hz, 1H), 4.62 - 4.32 (m, 3H), 3.97 (s, br, 1H), 3.37 - 3.24 (m, 3H), 3.21 - 3.06 (m, 2H), 2.54 - 2.44 (m, 2H), 2.26 - 2.11 (m, 2H), 1.08 (d, *J=* 6.7 Hz, 3H). MS (ESI) *m*/*z* 490 (M+H)⁺.

### Example 7

### 5-[(1S,3s)-3-{(3R)-4-[5-(2-hydroxypropan-2-yl)pyrimidin-2-yl]-3-methylpiperazine-1-carbonyl}cyclobutyl]-3-(trifluoromethyl)pyridin-2(1H)-one

### Example 7A

### methyl (R)-2-(4-(tert-butoxycarbonyl)-2-methylpiperazin-1-yl)pyrimidine-5-carboxylate

To a solution of methyl 2-chloropyrimidine-5-carboxylate (3.00 g, 17.4 mmol) in *N,N-*dimethylformamide (30 mL) was added potassium carbonate (7.21 g, 52.2 mmol) and (*R*)*-tert-*butyl 3-methylpiperazine-1-carboxylate (3.48 g, 17.4 mmol). The reaction mixture was stirred at 80 °C for 12 hours and filtered. The filtrate was slowly poured into ice water (500 mL). The material formed was collected via filtration. The filter cake was washed with water and dried under vacuum to provide the title compound.

### Example 7B

### tert-butyl (R)-4-(5-(2-hydroxypropan-2-yl)pyrimidin-2-yl)-3-methylpiperazine-1-carboxylate

To a solution of Example 7A (3.50 g, 10.4 mmol) in tetrahydrofuran (35 mL) was added methylmagnesium bromide solution (31.2 mL, 31.2 mmol) dropwise over 30 minutes at -78 °C under nitrogen. The reaction mixture was allowed to slowly warm up to 25 °C overnight. The reaction mixture was quenched with saturated aqueous ammonium chloride and was extracted with ethyl acetate three times. The combined organic layers were washed with saturated aqueous sodium chloride, dried with anhydrous sodium sulfate, and filtered. The filtrate was concentrated under vacuum. The residue was purified by flash chromatography (silica gel, petroleum ether/ethyl acetate = 100:0 to 35:65) to provide the title compound. MS (ESI) *m*/*z* 337 (M+H)⁺.

### Example 7C

### (R)-2-(2-(2-methylpiperazin-1-yl)pyrimidin-5-yl)propan-2-ol

A solution of Example 7B (2.60 g, 7.73 mmol) in dichloromethane (30 mL) was treated with trifluoroacetic acid (10 mL). The reaction mixture was stirred at room temperature for 30 minutes, quenched by addition of saturated aqueous sodium bicarbonate, and concentrated to remove the organic solvent. The aqueous residue was lyophilized to provide a crude product, which was purified by reversed phase HPLC (C18, 5 - 45% acetonitrile in water containing 10 mM ammonium bicarbonate) to provide the title compound. MS (ESI) *m*/*z* 237 (M+H)⁺.

### Example 7D

### 5-[(1S,3s)-3-{(3R)-4-[5-(2-hydroxypropan-2-yl)pyrimidin-2-yl]-3-methylpiperazine-1-carbonyl}cyclobutyl]-3-(trifluoromethyl)pyridin-2(1H)-one

Example 7D was prepared according to the procedure used for the preparation of Example 1E, substituting Example 7C for Example 1D. Purification by flash chromatography provided the title compound. ¹H NMR (400 MHz, DMSO-*d*₆, 90 °C) *δ* ppm 11.83 (s, br, 1H), 8.43 (s, 2H), 7.78 (d, *J* = 2.6 Hz, 1H), 7.42 (d, *J* = 2.6 Hz, 1H), 4.87 - 4.77 (m, 1H), 4.73 (s, 1H), 4.42 - 4.35 (m, 1H), 3.97 (s, br, 3H), 3.40 - 3.06 (m, 4H), 2.55 - 2.47 (m, 2H), 2.24 - 2.13 (m, 2H), 1.43 (s, 6H), 1.07 (d, *J =* 6.6 Hz, 3H). MS (ESI) *m*/*z* 462 (M-H₂O+H)⁺.

### Example 8

### 5-[(1R,3s)-3-{(3S)-3-methyl-4-[5-(trifluoromethyl)pyrimidin-2-yl]piperazine-1-carbonyl}cyclobutyl]-3-(trifluoromethyl)pyridin-2(1H)-one

### Example 8A

### (S)-tert-butyl 3-methyl-4-(5-(trifluoromethyl)pyrimidin-2-yl)piperazine-1-carboxylate

In Example 8A, (*S*)-*tert*-Butyl 3-methylpiperazine-1-carboxylate (4.00 g, 20.0 mmol), 2-chloro-5-(trifluoromethyl)pyrimidine (3.65 g, 20.0 mmol), and triethylamine (5.57 mL, 39.9 mmol) were combined in acetonitrile (80 mL). The reaction mixture was stirred at 80 °C for 4 hours and cooled to room temperature. The insoluble salt material was filtered. The filtrate was concentrated under vacuum. The residue was purified by flash chromatography (silica gel, 0-50% ethyl acetate in heptanes) to provide the title compound. MS (ESI) *m*/*z* 347 (M+H)⁺.

### Example 8B

### (S)-2-(2-methylpiperazin-1-yl)-5-(trifluoromethyl)pyrimidine

To a solution of Example 8A (5.80 g, 16.8 mmol) in acetonitrile (50 mL) was added 4M hydrogen chloride in dioxane (21 mL, 84 mmol). The reaction mixture was stirred at room temperature for 4 hours and filtered. The filter cake was rinsed with a small amount of acetonitrile and dried under vacuum to provide the title compound as a hydrochloric acid salt. The filtrate was concentrated and dried under vacuum to provide additional title compound as a hydrochloric acid salt. MS (ESI) *m*/*z* 247 (M+H)⁺.

### Example 8C

### 5-[(1R,3s)-3-{(3S)-3-methyl-4-[5-(trifluoromethyl)pyrimidin-2-yl]piperazine-1-carbonyl}cyclobutyl]-3-(trifluoromethyl)pyridin-2(1H)-one

Example 8C was prepared according to the procedure used for the preparation of Example 3C, substituting Example 8B for Example 3B, to provide the title compound. ¹H NMR (400 MHz, DMSO-*d*₆, 90 °C) δ ppm 11.83 (s, 1H), 8.66 (d, *J =* 0.8 Hz, 2H), 7.78 (d, *J* = 2.6 Hz, 1H), 7.43 (d, *J* = 2.6 Hz, 1H), 4.94 - 4.84 (m, 1H), 4.53 - 4.43 (m, 1H), 4.14 (s, br, 1H), 3.82 (s, br, 1H), 3.39 - 3.23 (m, 3H), 3.08 (s, br, 2H), 2.56 - 2.49 (m, 2H), 2.24 - 2.14 (m, 2H), 1.14 (d, *J* = 6.7 Hz, 3H). MS (ESI) *m*/*z* 490 (M+H)⁺.

### Example 9

### 5-[(1S,3s)-3-{(2R)-4-[5-(2-hydroxypropan-2-yl)pyrimidin-2-yl]-2-methylpiperazine-1-carbonyl}cyclobutyl]-3-(trifluoromethyl)pyridin-2(1H)-one

### Example 9A

### methyl (R)-2-(4-(tert-butoxycarbonyl)-3-methylpiperazin-1-yl)pyrimidine-5-carboxylate

Example 9A was prepared according to the procedure used for the preparation of Example 7A, substituting *tert*-butyl (*R*)-2-methylpiperazine-1-carboxylate for (*R*)-*tert*-butyl 3-methylpiperazine-1-carboxylate, to provide the title compound. ¹H NMR (400 MHz, DMSO-*d₆*) *δ* ppm 8.79 (s, 2H), 4.58 - 4.45 (m, 2H), 4.28 - 4.19 (m, 1H), 3.80 (s, 3H), 3.29 (br dd, *J =* 4.0, 13.4 Hz, 2H), 3.13 - 3.07 (m, 2H), 1.41 (s, 9H), 1.02 (d, *J* = 6.7 Hz, 3H).

### Example 9B

### tert-butyl (R)-4-(5-(2-hydroxypropan-2-yl)pyrimidin-2-yl)-2-methylpiperazine-1-carboxylate

Example 9B was prepared according to the procedure used for the preparation of Example 7B, substituting Example 9A for Example 7A, to provide the title compound. MS (ESI) *m*/*z* 337 (M+H)⁺.

### Example 9C

### (R)-2-(2-(3-methylpiperazin-1-yl)pyrimidin-5-yl)propan-2-ol

Example 9C was prepared according to the procedure used for the preparation of Example 7C, substituting Example 9B for Example 7B, to provide the title compound. MS (ESI) *m*/*z* 237 (M+H)⁺.

### Example 9D

### 5-[(1S,3s)-3-{(2R)-4-[5-(2-hydroxypropan-2-yl)pyrimidin-2-yl]-2-methylpiperazine-1-carbonyl}cyclobutyl]-3-(trifluoromethyl)pyridin-2(1H)-one

Example 9D was prepared according to the procedure used for the preparation of Example 1E, substituting Example 9C for Example 1D. Purification by flash chromatography provided the title compound. ¹H NMR (400 MHz, DMSO-*d*₆, 90 °C) *δ* ppm 11.83 (s, br, 1H), 8.42 (s, 2H), 7.77 (d, *J* = 2.6 Hz, 1H), 7.42 (d, *J* = 2.6 Hz, 1H), 4.73 (s, 1H), 4.57 - 4.29 (m, 3H), 3.96 (s, br, 2H), 3.38 - 3.06 (m, 4H), 2.53 - 2.44 (m, 2H), 2.25 - 2.10 (m, 2H), 1.43 (s, 6H), 1.09 (d, *J* = 6.7 Hz, 3H). MS (ESI) *m*/*z* 462 (M-H₂O+H)⁺.

### Example 10

### 5-[(1R,3s)-3-{(3S)-4-[5-(2-hydroxypropan-2-yl)pyrimidin-2-yl]-3-methylpiperazine-1-carbonyl}cyclobutyl]-3-(trifluoromethyl)pyridin-2(1H)-one

### Example 10A

### methyl (S)-2-(4-(tert-butoxycarbonyl)-2-methylpiperazin-1-yl)pyrimidine-5-carboxylate

Example 10A was prepared according to the procedure used for the preparation of Example 7A, substituting *tert*-butyl (*S*)-3-methylpiperazine-1-carboxylate for (*R*)-*tert*-butyl 3-methylpiperazine-1-carboxylate, to provide the title compound. MS (ESI) *m*/*z* 281 (M-56+H)⁺.

### Example 10B

### tert-butyl (S)-4-(5-(2-hydroxypropan-2-yl)pyrimidin-2-yl)-3-methylpiperazine-1-carboxylate

Example 10B was prepared according to the procedure used for the preparation of Example 7B, substituting Example 10A for Example 7A, to provide the title compound. MS (ESI) *m*/*z* 337 (M+H)⁺.

### Example 10C

### (,S)-2-(2-(2-methylpiperazin-1-yl)pyrimidin-5-yl)propan-2-ol

Example 10C was prepared according to the procedure used for the preparation of Example 7C, substituting Example 10B for Example 7B, to provide the title compound. MS (ESI) *m*/*z* 237 (M+H)⁺.

### Example 10D

### 5-[(1R,3s)-3-{(3S)-4-[5-(2-hydroxypropan-2-yl)pyrimidin-2-yl]-3-methylpiperazine-1-carbonyl}cyclobutyl]-3-(trifluoromethyl)pyridin-2(1H)-one

Example 10D was prepared according to the procedure used for the preparation of Example 1E, substituting Example 10C for Example 1D. Purification by flash chromatography provided the title compound. ¹H NMR (400 MHz, DMSO-*d*₆, 90 °C) *δ* ppm 11.84 (s, br, 1H), 8.43 (s, 2H), 7.78 (d, *J* = 2.6 Hz, 1H), 7.42 (d, *J* = 2.6 Hz, 1H), 4.88 - 4.78 (m, 1H), 4.73 (s, 1H), 4.43 - 4.35 (m, 1H), 3.97 (s, br, 3H), 3.40 - 3.04 (m, 4H), 2.55 - 2.45 (m, 2H), 2.25 - 2.12 (m, 2H), 1.43 (s, 6H), 1.07 (d, *J =* 6.6 Hz, 3H). MS (ESI) *m*/*z* 462 (M-H₂O+H)⁺.

### Example 11

### 5-[(1R,3s)-3-{(2S)-4-[5-(2-hydroxypropan-2-yl)pyrimidin-2-yl]-2-methylpiperazine-1-carbonyl}cyclobutyl]-3-(trifluoromethyl)pyridin-2(1H)-one

### Example 11A

### methyl (S)-2-(4-(tert-butoxycarbonyl)-3-methylpiperazin-1-yl)pyrimidine-5-carboxylate

Example 11A was prepared according to the procedure used for the preparation of Example 7A, substituting tert-butyl (S)-2-methylpiperazine-1-carboxylate for (*R*)*-tert*-butyl *3-*methylpiperazine-1-carboxylate, to provide the title compound. ¹H NMR (400 MHz, DMSO-*d₆*) *δ* ppm 8.79 (s, 2H), 4.59 - 4.45 (m, 2H), 4.24 (br d, *J* = 3.0 Hz, 1H), 3.80 (m, 4H), 3.31 - 3.26 (m, 1H), 3.09 (d, *J* = 9.5 Hz, 2H), 1.41 (s, 9H), 1.02 (d, *J* = 6.7 Hz, 3H).

### Example 11B

### tert-butyl (S)-4-(5-(2-hydroxypropan-2-yl)pyrimidin-2-yl)-2-methylpiperazine-1-carboxylate

Example 11B was prepared according to the procedure used for the preparation of Example 7B, substituting Example 11A for Example 7A, to provide the title compound. MS (ESI) *m*/*z* 337 (M+H)⁺.

### Example 11C

### (S)-2-(2-(3-methylpiperazin-1-yl)pyrimidin-5-yl)propan-2-ol

Example 11C was prepared according to the procedure used for the preparation of Example 7C, substituting Example 11B for Example 7B, to provide the title compound. MS (ESI) *m*/*z* 237 (M+H)⁺.

### Example 11D

### 5-[(1R,3s)-3-{(2S)-4-[5-(2-hydroxypropan-2-yl)pyrimidin-2-yl]-2-methylpiperazine-1-carbonyl}cyclobutyl]-3-(trifluoromethyl)pyridin-2(1H)-one

Example 11D was prepared according to the procedure used for the preparation of Example 1E, substituting Example 11C for Example 1D. Purification by flash chromatography provided the title compound. ¹H NMR (400 MHz, DMSO-*d*₆, 90 °C) *δ* ppm 11.85 (s, br, 1H), 8.42 (s, 2H), 7.77 (d, *J* = 2.6 Hz, 1H), 7.42 (d, *J* = 2.6 Hz, 1H), 4.73 (s, 1H), 4.57 - 4.27 (m, 3H), 3.97 (s, br, 2H), 3.51 - 3.41 (m, 1H), 3.35 - 3.08 (m, 3H), 2.53 - 2.44 (m, 2H), 2.25 - 2.10 (m, 2H), 1.43 (s, 6H), 1.09 (d, *J=* 6.6 Hz, 3H). MS (ESI) *m*/*z* 462 (M-H₂O+H)⁺.

### Example 12

### 5-{[(1S,3s)-3-{(2R)-2-methyl-4-[5-(trifluoromethyl)pyrimidin-2-yl]piperazine-1-carbonyl}cyclobutyl]methyl}-3-(trifluoromethyl)pyridin-2(1H)-one

Example 12 was prepared according to the procedure used for the preparation of Example 1E, substituting Example 13D for Example 1C, to provide the title compound. ¹H NMR (400 MHz, DMSO-*d*₆, 90 °C) *δ* ppm 11.79 (s, br, 1H), 8.64 (d, J = 0.9 Hz, 2H), 7.71 (d, J = 2.6 Hz, 1H), 7.39 (d, J = 2.6 Hz, 1H), 4.59 - 4.34 (m, 3H), 3.95 (s, br, 1H), 3.32 - 3.03 (m, 4H), 2.45 (d, J = 7.0 Hz, 2H), 2.42 - 2.34 (m, 1H), 2.25 - 2.15 (m, 2H), 1.93 - 1.79 (m, 2H), 1.06 (d, J = 6.7 Hz, 3H). MS (ESI) *m*/*z* 504 (M+H)⁺.

### Example 13

### 5-{[(1s,3s)-3-{(2R,6S)-2,6-dimethyl-4-[5-(trifluoromethyl)pyrimidin-2-yl]piperazine-1-carbonyl}cyclobutyl]methyl}-3-(trifluoromethyl)pyridin-2(1H)-one

### Example 13A

### methyl 3-((6-methoxy-5-(trifluoromethyl)pyridin-3-yl)methylene)cyclobutanecarboxylate

In Example 13A, 5-Bromo-2-methoxy-3-(trifluoromethyl)pyridine (1.02 g, 4.00 mmol), methyl 3-((4,4,5,5-tetramethyl-1,3,2-dioxaborolan-2-yl)methylene)cyclobutanecarboxylate (1.01 g, 4.00 mmol), Pd(dppf)Cl₂ CH₂Cl₂ ([1,1'-bis(diphenylphosphino)ferrocene]dichloropalladium(II), complex with dichloromethane, 163 mg, 0.200 mmol) and potassium carbonate (1.11 g, 8.00 mmol) were combined in a mixture of dioxane (9.0 mL) and water (3.0 mL). The reaction mixture was purged with nitrogen for 5 minutes, stirred at 90 °C for 16 hours, cooled to room temperature, and partitioned between ethyl acetate and water. The organic layer was dried with anhydrous sodium sulfate, treated with 3-mercaptopropyl functionalized silica gel, filtered, and the filtrate was concentrated under vacuum. The residue was purified by flash chromatography (silica gel, 0-20% ethyl acetate in heptanes) to provide the title compound. MS (ESI) *m*/*z* 302 (M+H)⁺.

### Example 13B

### (1r,3s)-methyl 3-((6-methoxy-5-(trifluoromethyl)pyridin-3-yl)methyl)cyclobutanecarboxylate

Example 13A (740 mg, 2.46 mmol) and tetrahydrofuran (10 mL) were added to 210 mg wet 5% Pd/C in a pressure reactor. The reaction mixture was stirred at 25 °C for 8 hours under hydrogen (50 psi), filtered, washed with tetrahydrofuran, and concentrated under vacuum. The residue was purified by flash chromatography (silica gel, 0-20% ethyl acetate in heptanes) to provide the title compound, which contained about 20% corresponding trans-isomer. MS (ESI) *m*/*z* 304 (M+H)⁺.

### Example 13C

### (1r,3s)-3-((6-methoxy-5-(trifluoromethyl)pyridin-3-yl)methyl)cyclobutanecarboxylic acid

To a solution of Example 13B (710 mg, 2.34 mmol) in a mixture of tetrahydrofuran (6.0 mL)/methanol (6.0 mL)/water (2.0 mL) was added sodium hydroxide (468 mg, 11.7 mmol). The reaction mixture was stirred at room temperature for 1 hour and diluted with water. The pH was adjusted to 4 with the addition of 1M aqueous HCl and extracted with ethyl acetate. The organic layer was washed with saturated aqueous sodium chloride, dried with anhydrous sodium sulfate, and filtered. The filtrate was concentrated under vacuum to provide the title compound which contained about 20% corresponding trans-isomer. MS (ESI) *m*/*z* 290 (M+H)⁺.

### Example 13D

### (1r,3s)-3-((6-oxo-5-(trifluoromethyl)-1,6-dihydropyridin-3-yl)methyl)cyclobutanecarboxylic acid

Example 13C (676 mg, 2.34 mmol), *para*-toluenesulfonic acid monohydrate (889 mg, 4.67 mmol), and lithium chloride (495 mg, 11.7 mmol) were combined in *N,N-*dimethylformamide (10 mL). The reaction mixture was stirred at 100 °C for 10 hours, cooled to room temperature, and partitioned between ethyl acetate and water. The organic layer was washed with saturated aqueous sodium chloride, dried with anhydrous sodium sulfate, and filtered. The filtrate was concentrated under vacuum to provide the tittle compound which contained about 20% corresponding trans-isomer. The material was further purified by chiral SFC (Chiralpak^{®} AD-H, 5 µm, 30 × 250 mm; mobile phase A: carbon dioxide; mobile phase B: isopropanol; flow rate: 80 g/minute). The first eluted fraction was collected to provide the title compound. MS (ESI) *m*/*z* 276 (M+H)⁺.

### Example 13E

### 2-((3R,5S)-3,5-dimethylpiperazin-1-yl)-5-(trifluoromethyl)pyrimidine

In Example 13E, a mixture of (2R,6S)-2,6-dimethylpiperazine (1.00 g, 8.76 mmol), 2-chloro-5-(trifluoromethyl)pyrimidine (1.60 g, 8.76 mmol) and triethylamine (4.88 mL, 35.0 mmol) were combined in acetonitrile (35 mL). The reaction mixture was stirred at room temperature overnight. The insoluble salt material was filtered and washed with a small amount of acetonitrile. The combined filtrate was concentrated under vacuum to provide the title compound. MS (ESI) *m*/*z* 261 (M+H)⁺.

### Example 13F

### 5-{[(1s,3s)-3-{(2R,6S)-2,6-dimethyl-4-[5-(trifluoromethyl)pyrimidin-2-yl]piperazine-1-carbonyl}cyclobutyl]methyl}-3-(trifluoromethyl)pyridin-2(1H)-one

Example 13D (27.5 mg, 0.100 mmol), 1-[bis(dimethylamino)methylene]-1*H*-1,2,3-triazolo[4,5*-b*]pyridinium 3-oxid hexafluorophosphate (76.0 mg, 0.200 mmol) and *N,N-*diisopropylethylamine (0.070 mL, 0.400 mmol) were combined in *N*,*N-*dimethylformamide (1.0 mL) and the reaction mixture was stirred at room temperature for 5 minutes. To the reaction mixture was added Example 13E (26.0 mg, 0.100 mmol), and the resulting mixture was stirred at 50 °C for 24 hours, cooled to room temperature, and purified by reverse phase HPLC (C18, 20-100% acetonitrile/ in water with 0.1% trifluoroacetic acid). The acetonitrile was removed by evaporation, and the mixture was neutralized with saturated aqueous sodium bicarbonate. The mixture was extracted with ethyl acetate, dried with anhydrous sodium sulfate, and filtered. The filtrate was concentrated under vacuum to provide the title compound. ¹H NMR (400 MHz, DMSO-*d*₆, 90 °C) *δ* ppm 11.79 (s, br, 1H), 8.64 (d, J = 0.8 Hz, 2H), 7.71 (d, J = 2.6, 1H), 7.39 (d, J = 2.6 Hz, 1H), 4.54 (d, J = 13.7 Hz, 2H), 4.36 (s, br, 2H), 3.26 - 3.12 (m, 3H), 2.46 (d, J = 7.3 Hz, 2H), 2.43 - 2.33 (m, 1H), 2.24 - 2.15 (m, 2H), 1.95 - 1.84 (m, 2H), 1.14 (d, J = 6.9 Hz, 6H). MS (ESI) *m*/*z* 518 (M+H)⁺.

### Example 14

### 5-{[(1s,3s)-3-{(3R,5S)-3,5-dimethyl-4-[5-(trifluoromethyl)pyrimidin-2-yl]piperazine-1-carbonyl}cyclobutyl]methyl}-3-(trifluoromethyl)pyridin-2(1H)-one

Example 14 was prepared according to the procedure used for the preparation of Example 1E, substituting Example 13D and Example 4B for Example 1C and Example 1D, respectively, to provide the title compound. ¹H NMR (400 MHz, DMSO-*d*₆, 90 °C) *δ* ppm 11.65 (s, br, 1H), 8.68 (d, *J* = 0.9 Hz, 2H), 7.71 (d, *J* = 2.6 Hz, 1H), 7.40 (d, *J* = 2.6 Hz, 1H), 4.87 - 4.78 (m, 2H), 3.98 (s, br, 2H), 3.42 - 2.90 (m, 3H), 2.47 (d, *J* = 7.5 Hz, 2H), 2.45 - 2.35 (m, 1H), 2.27 - 2.18 (m, 2H), 1.96 - 1.85 (m, 2H), 1.17 (d, *J* = 6.8 Hz, 6H). MS (ESI) *m*/*z* 518 (M+H)⁺.

### Example 15

### 5-{[(1S,3s)-3-{(3R)-3-methyl-4-[5-(trifluoromethyl)pyrimidin-2-yl]piperazine-1-carbonyl}cyclobutylmethyl}-3-(trifluoromethyl)pyridin-2(1H)-one

Example 15 was prepared according to the procedure used for the preparation of Example 1E, substituting Example 13D and Example 3B for Example 1C and Example 1D, respectively, to provide the title compound. ¹H NMR (400 MHz, DMSO-*d*₆, 90 °C) *δ* ppm 11.76 (s, br, 1H), 8.65 (d, *J* = 0.9 Hz, 2H), 7.71 (d, *J* = 2.6 Hz, 1H), 7.39 (d, *J* = 2.6 Hz, 1H), 4.93 - 4.81 (m, 1H), 4.51 - 4.41 (m, 1H), 3.97 (s, br, 2H), 3.38 - 3.00 (m, 4H), 2.45 (d, *J* = 7.7 Hz, 2H), 2.43 - 2.34 (m, 1H), 2.28 - 2.15 (m, 2H), 1.93 - 1.81 (m, 2H), 1.12 (d, *J* = 6.7 Hz, 3H). MS (ESI) *m*/*z* 504 (M+H)⁺.

### Example 16

### 5-{[(1R,3s)-3-{(3S)-3-methyl-4-[5-(trifluoromethyl)pyrimidin-2-yl]piperazine-1-carbonyl}cyclobutyl]methyl}-3-(trifluoromethyl)pyridin-2(1H)-one

Example 16 was prepared according to the procedure used for the preparation of Example 1E, substituting Example 13D and Example 8B for Example 1C and Example 1D, respectively, to provide the title compound. ¹H NMR (400 MHz, DMSO-*d*₆, 90 °C) *δ* ppm 11.78 (s, br, 1H), 8.65 (d, *J =* 0.9 Hz, 2H), 7.71 (d, *J = 2.6* Hz, 1H), 7.39 (d, *J =* 2.6 Hz, 1H), 4.92 - 4.82 (m, 1H), 4.49 - 4.42 (m, 1H), 3.98 (s, br, 2H), 3.35 - 3.01 (m, 4H), 2.45 (d, *J= 7.5* Hz, 2H), 2.43 - 2.34 (m, 1H), 2.28 - 2.15 (m, 2H), 1.93 - 1.82 (m, 2H), 1.12 (d, *J* = 6.7 Hz, 3H). MS (ESI) *m*/*z 504* (M+H)⁺.

### Example 17

### 5-{[(1R,3s)-3-{(2S)-2-methyl-4-[5-(trifluoromethyl)pyrimidin-2-yl]piperazine-1-carbonyl}cyclobutyl]methyl}-3-(trifluoromethyl)pyridin-2(1H)-one

Example 17 was prepared according to the procedure used for the preparation of Example 1E, substituting Example 13D and Example 6B for Example 1C and Example 1D, respectively, to provide the title compound. ¹H NMR (400 MHz, DMSO-*d*₆, 90 °C) *δ* ppm 11.78 (s, br, 1H), 8.64 (d, *J* = 0.9 Hz, 2H), 7.70 (d, *J* = 2.6 Hz, 1H), 7.39 (d, *J* = 2.6 Hz, 1H), 4.60 - 4.30 (m, 3H), 3.95 (s, br, 1H), 3.32 - 3.04 (m, 4H), 2.45 (d, *J* = 7.6 Hz, 2H), 2.42 - 2.33 (m, 1H), 2.24 - 2.16 (m, 2H), 1.93 - 1.79 (m, 2H), 1.05 (d, *J =* 6.7 Hz, 3H). MS (ESI) *m*/*z* 504 (M+H)⁺.

### Example 18

### 5-{[(1S,3s)-3-{(3R)-4-[5-(2-hydroxypropan-2-yl)pyrimidin-2-yl]-3-methylpiperazine-1-carbonyl}cyclobutyl]methyl}-3-(trifluoromethyl)pyridin-2(1H)-one

Example 18 was prepared according to the procedure used for the preparation of Example 1E, substituting Example 13D and Example 7C for Example 1C and Example 1D, respectively. Purification by flash chromatography provided the title compound. ¹H NMR (400 MHz, DMSO-*d*₆, 90 °C) *δ* ppm 11.79 (s, br, 1H), 8.42 (s, 2H), 7.71 (d, *J* = 2.6 Hz, 1H), 7.39 (d, *J* = 2.6 Hz, 1H), 4.86 - 4.66 (m, 2H), 4.41 - 4.33 (m, 1H), 3.94 (s, br, 2H), 3.29 - 3.02 (m, 4H), 2.45 (d, *J =* 7.2 Hz, 2H), 2.43 - 2.34 (m, 1H), 2.28 - 2.14 (m, 2H), 1.92 - 1.82 (m, 2H), 1.42 (s, 6H), 1.05 (d, *J* = 6.7 Hz, 3H). MS (ESI) *m*/*z* 476 (M-H₂O+H)⁺.

### Example 19

### 5-{[(1S,3s)-3-{(2R)-4-[5-(2-hydroxypropan-2-yl)pyrimidin-2-yl]-2-methylpiperazine-1-carbonyl}cyclobutyl]methyl}-3-(trifluoromethyl)pyridin-2(1H)-one

Example 19 was prepared according to the procedure used for the preparation of Example 1E, substituting Example 13D and Example 9C for Example 1C and Example 1D, respectively. Purification by flash chromatography provided the title compound. ¹H NMR (400 MHz, DMSO-*d*₆, 90 °C) *δ* ppm 11.78 (s, br, 1H), 8.41 (s, 2H), 7.70 (d, *J=* 2.6 Hz, 1H), 7.39 (d, *J=* 2.6 Hz, 1H), 4.73 (s, 1H), 4.55 - 4.21 (m, 3H), 3.95 (s, br, 1H), 3.22 - 2.85 (m, 4H), 2.45 (d, *J=* 7.4 Hz, 2H), 2.42 - 2.33 (m, 1H), 2.24 - 2.15 (m, 2H), 1.93 - 1.79 (m, 2H), 1.42 (s, 6H), 1.06 (d, *J=* 6.7 Hz, 3H). MS (ESI) *m*/*z* 476 (M-H₂O+H)⁺.

### Example 20

### 5-{[(1R,3s)-3-{(3S)-4-[5-(2-hydroxypropan-2-yl)pyrimidin-2-yl]-3-methylpiperazine-1-carbonyl}cyclobutyl]methyl}-3-(trifluoromethyl)pyridin-2(1H)-one

Example 20 was prepared according to the procedure used for the preparation of Example 1E, substituting Example 13D and Example 10C for Example 1C and Example 1D, respectively. Purification by flash chromatography provided the title compound. ¹H NMR (400 MHz, DMSO-*d*₆, 90 °C) *δ* ppm 11.79 (s, br, 1H), 8.42 (s, 2H), 7.71 (d, *J* = 2.6, 1H), 7.39 (d, *J =* 2.6 Hz, 1H), 4.86 - 4.66 (m, 2H), 4.41 - 4.33 (m, 1H), 3.95 (s, br, 2H), 3.29 - 3.00 (m, 4H), 2.45 (d, *J =* 7.2 Hz, 2H), 2.42 - 2.34 (m, 1H), 2.28 - 2.14 (m, 2H), 1.93 - 1.82 (m, 2H), 1.42 (s, 6H), 1.05 (d, *J=* 6.6 Hz, 3H). MS (ESI) *m*/*z* 476 (M-H₂O+H)⁺.

### Example 21

### 5-{[(1R,3s)-3-{(2S)-4-[5-(2-hydroxypropan-2-yl)pyrimidin-2-yl]-2-methylpiperazine-1-carbonyl}cyclobutyl]methyl}-3-(trifluoromethyl)pyridin-2(1H)-one

Example 21 was prepared according to the procedure used for the preparation of Example 1E, substituting Example 13D and Example 11C for Example 1C and Example 1D, respectively. Purification by flash chromatography provided the title compound. ¹H NMR (400 MHz, DMSO-*d*₆, 90 °C) *δ* ppm 11.78 (s, br, 1H), 8.41 (s, 2H), 7.71 (d, *J* = 2.6 Hz, 1H), 7.39 (d, *J =* 2.6 Hz, 1H), 4.73 (s, 1H), 4.54 - 4.23 (m, 3H), 3.96 (s, br, 1H), 3.22 - 2.85 (m, 4H), 2.45 (d, *J =* 7.3 Hz, 2H), 2.42 - 2.33 (m, 1H), 2.24 - 2.15 (m, 2H), 1.94 - 1.79 (m, 2H), 1.42 (s, 6H), 1.06 (d, *J* = 6.7 Hz, 3H). MS (ESI) *m*/*z* 476 (M-H₂O+H)⁺.

### Example 22

### 5-{[(1s,3s)-3-{(3R,5S)-4-[5-(2-hydroxypropan-2-yl)pyrimidin-2-yl]-3,5-dimethylpiperazine-1-carbonyl}cyclobutyl]methyl}-3-(trifluoromethyl)pyridin-2(1H)-one

### Example 22A

### methyl 2-((2S,6R)-4-(tert-butoxycarbonyl)-2,6-dimethylpiperazin-1-yl)pyrimidine-5-carboxylate

In Example 22A, methyl 2-chloropyrimidine-5-carboxylate (1.50 g, 8.69 mmol) and *tert-*butyl (3*S*,5*R*)-3,5-dimethylpiperazine-1-carboxylate (3.73 g, 17.4 mmol) were combined in *N-*methyl-2-pyrrolidinone (20 mL). The reaction mixture was stirred at 140 °C for 12 hours, cooled to room temperature, diluted with water, and extracted with ethyl acetate three times. The combined organic layers were concentrated under vacuum. The residue was purified by flash chromatography (silica gel, 0-100% ethyl acetate in petroleum ether) to provide the title compound. MS (ESI) *m*/*z* 351 (M+H)⁺.

### Example 22B

### tert-butyl (3S,5R)-4-(5-(2-hydroxypropan-2-yl)pyrimidin-2-yl)-3,5-dimethylpiperazine-1-carboxylate

To a solution of Example 22A (1.60 g, 4.57 mmol) in tetrahydrofuran (15 mL) at -78 °C under nitrogen was added 3.0 M methylmagnesium bromide (7.61 mL, 22.8 mmol). The reaction mixture was stirred at room temperature for 2 hours, quenched with aqueous ammonium chloride, diluted with water, and extracted with ethyl acetate three times. The combined organic layers were washed with saturated aqueous sodium chloride, dried with anhydrous sodium sulfate, and filtered. The filtrate was concentrated under vacuum. The residue was purified by flash chromatography (silica gel, 0-50% ethyl acetate in petroleum ether) to provide the title compound. MS (ESI) *m*/*z* 351 (M+H)⁺.

### Example 22C

### 2-(2-((2S,6R)-2,6-dimethylpiperazin-1-yl)pyrimidin-5-yl)propan-2-ol

A mixture of Example 22B (800 mg, 2.28 mmol) in 4 M HCl in ethyl acetate (2 mL) was stirred at room temperature for 2 hours and concentrated under vacuum. The pH was adjusted to about 8 by progressively adding saturated aqueous sodium bicarbonate. The mixture was purified by preparative reversed phase HPLC (C18, 5 - 95% acetonitrile in water) to provide the title compound. MS (ESI) *m*/*z* 251 (M+H)⁺.

### Example 22D

### 5-{[(1s,3s)-3-{(3R,5S)-4-[5-(2-hydroxypropan-2-yl)pyrimidin-2-yl]-3,5-dimethylpiperazine-1-carbonyl}cyclobutyl]methyl}-3-(trifluoromethyl)pyridin-2(1H)-one

Example 22D was prepared according to the procedure used for the preparation of Example 1E, substituting Example 13D and Example 22C for Example 1C and Example 1D, respectively. Purification by flash chromatography provided the title compound. ¹H NMR (400 MHz, DMSO-*d*₆, 90 °C) *δ* ppm 11.78 (s, br, 1H), 8.44 (s, 2H), 7.71 (d, *J* = 2.6 Hz, 1H), 7.40 (d, *J* = 2.6 Hz, 1H), 4.80 - 4.67 (m, 3H), 3.95 (s, br, 2H), 3.34 - 3.02 (m, 3H), 2.46 (d, *J* = 7.5 Hz, 2H), 2.44 - 2.35 (m, 1H), 2.27 - 2.18 (m, 2H), 1.96 - 1.86 (m, 2H), 1.43 (s, 6H), 1.12 (d, *J=* 6.8 Hz, 6H). MS (ESI) *m*/*z* 490 (M-H₂O+H)⁺.

### Example 23

### 3-(trifluoromethyl)-5-[(1s,3s)-3-{4-[5-(trifluoromethyl)pyrimidin-2-yl]piperazine-1-carbonyl}cyclobutyl]pyridin-2(1H)-one

### Example 23A

### methyl 3-(6-methoxy-5-(trifluoromethyl)pyridin-3-yl)cyclobutanecarboxylate

Example 23A was prepared according to the procedure used for the preparation of Example 1A. Purification provided the title compound as the mixture of stereoisomers with both trans configuration and cis configuration. MS (ESI) *m*/*z* 290 (M+H)⁺.

### Example 23B

### 3-(6-methoxy-5-(trifluoromethyl)pyridin-3-yl)cyclobutanecarboxylic acid

Example 23B was prepared according to the procedure used for the preparation of Example 1B, substituting Example 23A for Example 1A, to provide the title compound. MS (ESI) *m*/*z* 276 (M+H)⁺.

### Example 23C

### tert-butyl 4-(5-(trifluoromethyl)pyrimidin-2-yl)piperazine-1-carboxylate

In Example 23C, a 4-neck 1-L jacketed reactor was equipped with overhead stirring, a Huber temperature probe, and a nitrogen inlet. Under a nitrogen atmosphere, the reactor was charged with 2-chloro-5-(trifluoromethyl)pyrimidine (59.6 g, 326 mmol), which was dissolved in acetonitrile (600 mL). The solution was cooled to 5 °C internal and then *tert*-butyl piperazine-1-carboxylate (60.8 g, 326 mmol) was added followed by *N,N-*diisopropylethylamine (142 mL, 816 mmol). The reaction mixture was stirred at room temperature for 1 hour, transferred to a round bottom flask, and concentrated under vacuum. The residue was dissolved in dichloromethane, washed with saturated aqueous ammonium chloride, washed with saturated aqueous sodium chloride, dried with anhydrous magnesium sulfate, and filtered. The filtrate was concentrated under vacuum to provide the title compound. MS (APCI) *m*/*z* 277 (M-56+H)⁺.

### Example 23D

### 2-(piperazin-1-yl)-5-(trifluoromethyl)pyrimidine

In Example 23D, a 4-neck 2-L jacketed reactor was equipped with overhead stirring, a Huber temperature probe and a nitrogen inlet. Under a nitrogen atmosphere, the reactor was charged with Example 23C (108 g, 325 mmol), followed by addition of dioxane (1000 mL) and 4M HCl (300 mL, 1200 mmol). The reaction mixture was stirred at room temperature for 18 hours, filtered, rinsed with *tert*-butyl methyl ether, and dried under vacuum. The residue was suspended in dichloromethane and cooled in an ice bath. Saturated aqueous sodium bicarbonate was added slowly until a neutral pH was reached, and the mixture was separated. The aqueous layer was extracted with dichloromethane twice. The combined organic layers were washed with saturated aqueous sodium chloride, dried with anhydrous magnesium sulfate, and filtered. The filtrate was concentrated under vacuum to provide the title compound. To the aqueous layer was added NaOH pellets to adjust the pH > 10, and the mixture was extracted with dichloromethane three times. The combined organic layers were washed with saturated aqueous sodium chloride, dried with anhydrous magnesium sulfate, and filtered. The filtrate was concentrated under vacuum to provide additional title compound. MS (APCI) *m*/*z* 233 (M+H)⁺.

### Example 23E

### (3-(6-methoxy-5-(trifluoromethyl)pyridin-3-yl)cyclobutyl)(4-(5-(trifluoromethyl)pyrimidin-2-yl)piperazin-1-yl)methanone

To a mixture of Example 23B (84.0 mg, 0.305 mmol), Example 23D (70.9 mg, 0.305 mmol), and *N,N*-diisopropylethylamine (0.320 mL, 1.83 mmol) in *N,N-*dimethylformamide (2.0 mL) was added 50% propanephosphonic acid anhydride in *N,N-*dimethylformamide (388 mg, 0.610 mmol). The reaction mixture was stirred at room temperature for 1 hour and partitioned with ethyl acetate and water. The organic layer was washed with saturated aqueous sodium chloride, dried with anhydrous sodium sulfate, and filtered. The filtrate was concentrated under vacuum to provide the title compound. MS (ESI) *m*/*z* 490 (M+H)⁺.

### Example 23F

### 3-(trifluoromethyl)-5-[(1s,3s)-3-{4-[5-(trifluoromethyl)pyrimidin-2-yl]piperazine-1-carbonyl}cyclobutyl]pyridin-2(1H)-one

Example 23E (134 mg, 0.274 mmol), *para*-toluenesulfonic acid monohydrate (104 mg, 0.548 mmol), and lithium chloride (58.0 mg, 1.37 mmol) were combined in *N,N-*dimethylformamide (2.0 mL). The reaction mixture was stirred at 100 °C for 10 hours, cooled to room temperature, and purified by reverse phase HPLC (C18, 10-100% acetonitrile/ in water with 0.1% trifluoroacetic acid). The acetonitrile was removed by evaporation under vacuum, and the resulting mixture was neutralized with saturated aqueous sodium bicarbonate. The resulting mixture was extracted with ethyl acetate, dried with anhydrous sodium sulfate, and filtered. The filtrate was concentrated under vacuum. The residue was further purified by flash chromatography to provide the two fractions. The first eluted fraction was the stereoisomer with trans-configuration. The second eluted fraction was the title compound with cis-configuration. ¹H NMR (500 MHz, DMSO-*d*₆) *δ* ppm 12.21 (s, 1H), 8.73 (d, *J =* 0.8 Hz, 2H), 7.83 (d, *J =* 2.6 Hz, 1H), 7.50 (d, *J =* 2.6 Hz, 1H), 3.86 - 3.79 (m, 4H), 3.58 - 3.54 (m, 2H), 3.52 - 3.48 (m, 2H), 3.37 - 3.24 (m, 2H), 2.49 - 2.41 (m, 2H), 2.23 - 2.13 (m, 2H). MS (ESI) *m*/*z* 476 (M+H)⁺.

### Example 24

### 5-[(1r,3r)-3-{(3R,5S)-3,5-dimethyl-4-[5-(trifluoromethyl)pyrimidin-2-yl]piperazine-1-carbonyl}cyclobutyl]-3-(trifluoromethyl)pyridin-2(1H)-one

Example 24 was prepared according to the procedure used for the preparation of Example 1E, substituting Example 25C and Example 4B for Example 1C and Example 1D, respectively, to provide the title compound. ¹H NMR (400 MHz, DMSO-*d*₆, 90 °C) *δ* ppm 11.61 (s, 1H), 8.68 (d, *J* = 0.9 Hz, 2H), 7.85 (d, *J =* 2.6 Hz, 1H), 7.51 (d, *J =* 2.6 Hz, 1H), 4.89 - 4.79 (m, 2H), 3.86 (s, br, 2H), 3.52 - 3.36 (m, 2H), 3.30 - 3.02 (m, 2H), 2.68 - 2.51 (m, 2H), 2.34 - 2.24 (m, 2H), 1.19 (d, *J* = 6.9 Hz, 6H). MS (ESI) *m*/*z* 504 (M+H)⁺.

### Example 25

### 5-[(1R,3r)-3-{(2R)-2-methyl-4-[5-(trifluoromethyl)pyrimidin-2-yl]piperazine-1-carbonyl}cyclobutyl]-3-(trifluoromethyl)pyridin-2(1H)-one

### Example 25A

### (1r,3r)-methyl 3-(6-methoxy-5-(trifluoromethyl)pyridin-3-yl)cyclobutanecarboxylate

Example 25A was prepared as described in Example 1A. The first eluted fraction was the title compound with trans configuration. MS (ESI) *m*/*z* 290 (M+H)⁺.

### Example 25B

### (1r,3r)-3-(6-methoxy-5-(trifluoromethyl)pyridin-3-yl)cyclobutanecarboxylic acid

Example 25B was prepared according to the procedure used for the preparation of Example 1B, substituting Example 25A for Example 1A, to provide the title compound. MS (ESI) *m*/*z* 276 (M+H)⁺.

### Example 25C

### (1r,3r)-3-(6-oxo-5-(trifluoromethyl)-1,6-dihydropyridin-3-yl)cyclobutanecarboxylic acid

Example 25C was prepared according to the procedure used for the preparation of Example 1C, substituting Example 25B for Example 1B, to provide the title compound. MS (ESI) *m*/*z* 262 (M+H)⁺.

### Example 25D

### 5-[(1R,3r)-3-{(2R)-2-methyl-4-[5-(trifluoromethyl)pyrimidin-2-yl]piperazine-1-carbonyl}cyclobutyl]-3-(trifluoromethyl)pyridin-2(1H)-one

Example 25D was prepared according to the procedure used for the preparation of Example 1E, substituting Example 25C for Example 1C, to provide the title compound. ¹H NMR (400 MHz, DMSO-*d*₆, 90 °C) *δ* ppm 11.87 (s, 1H), 8.65 (d, *J* = 0.8 Hz, 2H), 7.84 (d, *J* = 2.6 Hz, 1H), 7.49 (d, *J* = 2.7 Hz, 1H), 4.69 - 4.27 (m, 3H), 3.82 (s, br, 1H), 3.42 - 3.28 (m, 3H), 3.26 - 3.03 (m, 2H), 2.64 - 2.50 (m, 2H), 2.31 - 2.20 (m, 2H), 1.08 (d, *J* = 6.7 Hz, 3H). MS (ESI) *m*/*z* 490 (M+H)⁺.

### Example 26

### 3-(trifluoromethyl)-5-[(1r,3r)-3-{4-[5-(trifluoromethyl)pyrimidin-2-yl]piperazine-1-carbonyl}cyclobutyl]pyridin-2(1H)-one

Example 26 was prepared as described in Example 23F. The first eluted fraction was the title compound with trans configuration. ¹H NMR (600 MHz, DMSO-*d*₆) *δ* ppm 12.22 (s, 1H), 8.73 (d, *J* = 0.9 Hz, 2H), 7.93 (d, *J* = 2.6 Hz, 1H), 7.58 (d, *J* = 2.6 Hz, 1H), 3.86 - 3.81 (m, 4H), 3.62 - 3.58 (m, 2H), 3.44 - 3.34 (m, 4H), 2.55 - 2.51 (m, 2H), 2.31 - 2.23 (m, 2H). MS (ESI) *m*/*z* 476 (M+H)⁺.

### Example 27

### 5-{[(1s,3s)-3-{4-[5-(2-hydroxypropan-2-yl)pyrimidin-2-yl]piperazine-1-carbonyl}cyclobutyl]methyl}-3-(trifluoromethyl)pyridin-2(1H)-one

### Example 27A

### 2-(2-(piperazin-1-yl)pyrimidin-5-yl)propan-2-ol

In Example 27A, 2-(2-Chloropyrimidin-5-yl)propan-2-ol (1.00 g, 5.79 mmol), piperazine (1.50 g, 17.4 mmol), and *N,N-*diisopropylethylamine (5.06 mL, 29.0 mmol) were combined in acetonitrile (10 mL). The reaction mixture was stirred at 80 °C for 2 hours, cooled to room temperature, and concentrated under vacuum. The residue was purified by flash chromatography to provide the title compound. MS (ESI) *m*/*z* 223 (M+H)⁺.

### Example 27B

### 5-{[(1s,3s)-3-{4-[5-(2-hydroxypropan-2-yl)pyrimidin-2-yl]piperazine-1-carbonyl}cyclobutyl]methyl}-3-(trifluoromethyl)pyridin-2(1H)-one

Example 27B was prepared according to the procedure used for the preparation of Example 1E, substituting Example 13D and Example 27A for Example 1C and Example 1D, respectively. Purification by flash chromatography provided the title compound. ¹H NMR ppm (400 MHz, DMSO-*d*₆) *δ* 12.15 (s, 1H), 8.44 (s, 2H), 7.80 (d, *J* = 2.5 Hz, 1H), 7.48 (d, *J* = 2.5 Hz, 1H), 5.08 (s, 1H), 3.71 - 3.63 (m, 4H), 3.53 - 3.46 (m, 2H), 3.43 - 3.36 (m, 2H), 3.24 - 3.11 (m, 1H), 2.43 (d, *J* = 7.3 Hz, 2H), 2.40 - 2.30 (m, 1H), 2.20 - 2.10 (m, 2H), 1.89 - 1.79 (m, 2H), 1.41 (s, 6H). MS (ESI) *m*/*z* 462 (M-H₂O+H)⁺.

### Example 28

### 5-[(1s,3s)-3-{4-[5-(2-hydroxypropan-2-yl)pyrimidin-2-yl]piperazine-1-carbonyl}cyclobutyl]-3-(trifluoromethyl)pyridin-2(1H)-one

Example 28 was prepared according to the procedure used for the preparation of Example 1E, substituting Example 27A for Example 1D. The reaction mixture was purified by reverse phase HPLC (C18, 20-100% acetonitrile/water with 0.1% trifluoroacetic acid). The acetonitrile was removed by evaporation under vacuum, and the resulting mixture was neutralized with saturated aqueous sodium bicarbonate, extracted with ethyl acetate, dried with anhydrous sodium sulfate, and filtered. The filtrate was concentrated under vacuum. The residue was further purified by flash chromatography to provide the title compound. ¹H NMR (400 MHz, DMSO-*d*₆) *δ* ppm 12.20 (s, 1H), 8.45 (s, 2H), 7.83 (d, *J =* 2.6 Hz, 1H), 7.50 (d, *J =* 2.6 Hz, 1H), 5.08 (s, 1H), 3.75 - 3.65 (m, 4H), 3.55 - 3.49 (m, 2H), 3.47 - 3.42 (m, 2H), 3.38 - 3.23 (m, 2H), 2.48 - 2.40 (m, 2H), 2.24 - 2.11 (m, 2H), 1.41 (s, 6H). MS (ESI) *m*/*z* 448 (M-H₂O+H)⁺.

### Example 29

### 3-(trifluoromethyl)-5-{[(1s,3s)-3-{4-[5-(trifluoromethyl)pyrimidin-2-yl]piperazine-1-carbonyl}cyclobutyl]methyl}pyridin-2(1H)-one

Example 29 was prepared according to the procedure used for the preparation of Example 1E, substituting Example 13D and Example 23D for Example 1C and Example 1D, respectively, to provide the title compound. ¹H NMR (600 MHz, DMSO-*d*₆) *δ* ppm 12.15 (s, br, 1H), 8.72 (d, *J* = 0.8 Hz, 2H), 7.80 (d, *J* = 2.5 Hz, 1H), 7.49 (d, *J* = 2.5 Hz, 1H), 3.83 - 3.77 (m, 4H), 3.56 - 3.51 (m, 2H), 3.47 - 3.41 (m, 2H), 3.22 - 3.15 (m, 1H), 2.43 (d, *J* = 7.5 Hz, 2H), 2.40 - 2.30 (m, 1H), 2.20 - 2.12 (m, 2H), 1.89 - 1.80 (m, 2H). MS (ESI) *m*/*z* 490 (M+H)⁺.

### TIPARP GST-(TEV) FUSION PROTEIN EXPRESSION AND PURIFICATION

### Expression

The full-length cDNA encoding the human TIPARP (NP_001171647.1, UniProtKB - Q7Z3E1) of 657 amino acids was the template to generate the expression construct. The nucleotide sequences were synthesized with codon optimization for expression in Sf9 insect cells. The TIPARP fusion protein includes GST, glutathione S-transferase, a 26 kDa protein whose DNA sequence is frequently integrated into expression vectors for production of recombinant proteins.

A truncated TIPARP protein sequence, consisting of amino acids 441 to 657, was cloned into vector pFastBac^{™}1 (Thermo Fisher Scientific) with an N-terminal GST tag followed by a TEV protease site to produce the TIPARP fusion protein, TIPARP GST-(TEV).

The TIPARP fusion protein was heterologously expressed in Sf9 insect cell cultures seeded with baculovirus-infected insect cells (BIICs) infected with the pFastBac^{™}1recombinant baculovirus. The baculovirus-infected cell cultures were grown in a Wave Bioreactor (Cytiva, Marlborough, MA, USA) on a 20-liter scale. The Sf9 insect cells were grown in Sf-900^{™} II SFM media (Thermo Fisher Scientific, Waltham, MA, USA) to a density of 2.4 x 10⁶ cells/mL before infection with baculovirus. For the infection, BIICs were added, and cells were harvested after 72 hours of incubation by centrifugation at 4,000 x g for 10 minutes at 5 °C and transferred to -80 °C until further processing.

The TIPARP fusion protein expressed in insect cells was purified using the following protocol: cells were thawed, homogenized and lysed in 20 mM HEPES (4-(2-hydroxyethyl)-1-piperazineethanesulfonic acid), pH 7.5, 250 mM NaCl, 5% glycerol (w/v), 0.1 % Triton X-100, 0.5 mM MgCl₂, 0.5 mM Tris (2-carboxyethyl) phosphine hydrochloride (TCEP), using a Dounce homogenizer. Benzonase^{®} Nuclease (MilliporeSigma, Burlington, MA, USA) and SIGMA*FAST*^{™} Protease Inhibitor Cocktail Tablets (MilliporeSigma) were added to the lysate according to the manufacturer's recommendations. The cell lysate was clarified by centrifugation at 35,000 x g for 60 minutes at 5 °C. Supernatant was sterile filtered through a 0.45 µm filter.

### Purification

The TIPARP fusion protein was purified from the supernatant in batch mode using Glutathione Sepharose 4 Fast Flow GST-tagged protein purification resin (Cytiva) equilibrated in column buffer (20 mM HEPES pH 7.5, 150 mM NaCl, 5% glycerol (v/v), 0.5 mM TCEP). Supernatant and resin were gently mixed and allowed to settle into a column by gravity flow. Resin was washed with 20 column volumes of column buffer (20 mM HEPES pH 7.5, 150 mM NaCl, 5% glycerol, 0.5 mM TCEP). Protein was eluted from the column using column buffer with the addition of 10 mM glutathione. Protein was further diluted 3-fold with 20 mM MES (2-(N-morpholino)ethanesulfonic acid) pH 6.1, 5% glycerol (v/v), and 0.5 mM TCEP buffer and loaded onto HiTrap^{®} SP Sepharose^{™} Fast Flow resin (MilliporeSigma) equilibrated in 20 mM MES pH 6.1, 50 mM NaCl, 5% glycerol, and 0.5 mM TCEP. The TIPARP fusion protein was eluted with a sodium chloride gradient from 0.05 M to 1 M in 10 column volumes, with the fusion protein eluting at 400 mM NaCl. The molecular weight of the purified fusion protein was confirmed by mass spectroscopy.

### SYNTHESIS OF PROBE 1

### Probe 1

### N-{2-[2-(2-{[1-(5-cyanopyridin-2-yl)-4-{3-[(4-oxo-3,4-dihydrophthalazin-1-yl)methyl]benzoyl}piperazin-2-yl]methoxy}ethoxy)ethoxy]ethyl}-2',7'-difluoro-3',6'-dihydroxy-3-oxo-3H-spiro[[2]benzofuran-1,9'-xanthene]-5-carboxamide

### Probe 1A

### 3-[(E)-(3-oxo-2-benzofuran-1(3H)-ylidene)methyl[benzonitrile

To a mixture of isobenzofuran-1(3*H*)-one (5 g, 37.3 mmol) and 3-formylbenzoic acid (4.89 g, 37.3 mmol) in ethyl acetate (20 mL) was added sodium methoxide (30.2 g, 168 mmol) dropwise at 0 °C. Methanol (10 mL) was added, and the mixture was stirred at 80 °C for 1 hour. LCMS showed that the starting material was consumed. The solvent was evaporated, and the residue was diluted with water (1.5 L). The mixture was acidified using 2 N aqueous hydrochloric acid at 20 °C to give pH = 1~2. The mixture was filtered, and the filter cake was washed with water (500 mL), and dried under high vacuum to give the title compound. ¹H NMR (400 MHz, DMSO-*d*₆) *δ* ppm 7.45 - 7.61 (m, 7H) 8.24 - 8.36 (m, 2H). ESI-MS *m*/*z* 246.0 (M-H).

### Probe 1B

### 3-[(4-oxo-3,4-dihydrophthalazin-1-yl)methyl]benzoic acid

To a mixture of 3-[(*E*)-(3-oxo-2-benzofuran-1(3*H*)-ylidene)methyl]benzonitrile (6 g, 24.27 mmol) in water (36 mL) was added a solution of sodium hydroxide (5.82 g, 146 mmol) in water (7.2 mL) at 20 °C. The reaction mixture was warmed to 90 °C and stirred for 1 hour. The reaction mixture was cooled to 70 °C and hydrazine (10.88 mL, 340 mmol) was added. The resulting mixture was stirred at 70 °C for another 18 hours. Thin layer chromatography (tetrahydrofuran/petroleum ether = 1/1) showed the starting material was consumed and a new spot was formed. The mixture was extracted with ethyl acetate (3 × 60 mL). The organic phase was discarded, and the aqueous phase was acidified with 1 N aqueous hydrochloric acid at 20 °C to pH = 4. The precipitate was collected by filtration. The filter cake was washed with water (60 mL) and petroleum ether (60 mL), and dried under high vacuum to give the title compound. ¹H NMR (400 MHz, DMSO-*d*₆) *δ* ppm 4.38 (s, 2H) 7.42 (t, J=7.70 Hz, 1H) 7.58 (br d, J=7.58 Hz, 1H) 7.80 (br dd, J=16.44, 7.89 Hz, 2H) 7.85 - 7.93 (m, 2H) 7.93 - 7.99 (m, 1H) 8.26 (d, J=7.58 Hz, 1H) 12.60 (s, 1H) 12.67 - 13.07 (m, 1H). ESI-MS *m*/*z* 281.1 (M+H)⁺.

### Probe 1C

### tert-butyl 4-(5-cyanopyridin-2-yl)-3-(hydroxymethyl)piperazine-1-carboxylate

In Probe 1C, *tert-*Butyl 3-(hydroxymethyl)piperazine-1-carboxylate (300 mg, 1.387 mmol) was combined with 6-chloronicotinonitrile (192 mg, 1.387 mmol) in dimethylacetamide (4 mL). *N,N*-Diisopropylethylamine (0.727 mL, 4.16 mmol) was added and the reaction was heated at 90 °C for 2.25 hours. The reaction mixture was cooled, diluted with water, and extracted with ethyl acetate. The organic layer was dried over sodium sulfate and filtered. The filtrate was concentrated, and the residue was purified using a silica gel cartridge and was eluted with 0-40% ethyl acetate/heptanes to provide the title compound. ¹H NMR (600 MHz, CDCl₃) *δ* ppm 8.40 (dd, *J* = 2.4, 0.8 Hz, 1H), 7.65 (dd, *J* = 9.0, 2.3 Hz, 1H), 6.65 (d, *J =* 9.0 Hz, 1H), 4.60 (s, 2H), 4.27 (s, 1H), 4.06 (d, *J =* 12.6 Hz, 1H), 3.98 (s, 1H), 3.63 (s, 1H), 3.49 (s, 2H), 3.33 (s, 1H), 3.27 (td, *J* = 12.2, 3.7 Hz, 1H), 3.16 (s, 1H), 1.50 (s, 8H). ESI-MS *m*/*z* 219.67 (M+H-Boc)⁺.

### Probe 1D

### tert-butyl 3-({2-[2-(2-azidoethoxy)ethoxy]ethoxy}methyl)-4-(5-cyanopyridin-2-yl)piperazine-1-carboxylate

In Probe 1D, *tert*-Butyl 4-(5-cyanopyridin-2-yl)-3-(hydroxymethyl)piperazine-1-carboxylate (58.3 mg, 0.183 mmol) and 2-(2-(2-azidoethoxy)ethoxy)ethyl methanesulfonate (55.7 mg, 0.220 mmol) were combined in anhydrous *N,N*-dimethylformamide (1 mL). Sodium hydride (60% in mineral oil, 11 mg, 0.275 mmol) was added and the mixture was stirred at ambient temperature for 1 hour, and then at 50 °C for 2 hours. Additional 2-(2-(2-azidoethoxy)ethoxy)ethyl methanesulfonate (55.7 mg, 0.220 mmol) was added and the reaction mixture was stirred at 50 °C for 2 hours. The reaction mixture was diluted to 3 mL using 90% DMSO/water and was purified in one injection on a Gilson^{®} RP-HPLC running uniPoint software with a Waters^{™} Deltapak C18 200 x 25 mm column (15 µm particle size, 100 Angstrom porosity) and was eluted with a gradient of 5% A (0.1% TFA-water):B (acetonitrile) to 100% A:B [0-5 minutes: 5% A; 5-45 minute linear gradient to 100% B 2.375%/minute gradient] with a 20 mL/minute flowrate. Fractions containing the title compound were combined and lyophilized to give the title compound as the trifluoroacetate salt. ESI-MS *m*/*z* 476.1 (M+H)⁺.

### Probe 1E

### 6-[2-({2-[2-(2-azidoethoxy)ethoxy]ethoxy}methyl)piperazin-1-yl]pyridine-3-carbonitrile

In Probe 1E, *tert*-Butyl 3-({2-[2-(2-azidoethoxy)ethoxy]ethoxy}methyl)-4-(5-cyanopyridin-2-yl)piperazine-1-carboxylate trifluoroacetate salt (47.2 mg, 0.080 mmol) was dissolved in 1 mL trifluoroacetic acid and immediately evaporated to dryness under a stream of dry nitrogen gas to give the title compound as the bis trifluroacetate salt. ESI-MS *m*/*z* 376.16 (M+H)⁺.

### Probe 1F

### 6-[2-({2-[2-(2-azidoethoxy)ethoxy]ethoxy}methyl)-4-({3-[(4-oxo-3,4-dihydrophthalazin-1-yl)methyl]phenyl}methyl)piperazin-1-yl]pyridine-3-carbonitrile

In Probe 1F, 6-[2-({2-[2-(2-Azidoethoxy)ethoxy]ethoxy}methyl)piperazin-1-yl]pyridine-3-carbonitrile bis trifluoroacetate salt (48.2 mg, 0.080 mmol) and 3-[(4-oxo-3,4-dihydrophthalazin-1-yl)methyl]benzoic acid (24.63 mg, 0.088 mmol) were combined in anhydrous *N,N*-dimethylformamide (1 mL). PyAOP ((7-azabenzotriazol-1-yloxy)tripyrrolidinophosphonium hexafluorophosphate, 45.8 mg, 0.088 mmol) and *N,N-*diisopropylethylamine (69.7 µl, 0.399 mmol) were added and the reaction was shaken at ambient temperature for one hour. An additional 24 mg PyAOP was added, and the reaction mixture was shaken for an additional hour at ambient temperature. The reaction mixture was diluted to 3 mL with 90% DMSO/water and was purified in one injection on a Gilson^{®} RP-HPLC running uniPoint software with a Waters^{™} Deltapak C18 200 x 25 mm column (15 um particle size, 100 Angstrom porosity) and was eluted with a gradient of 5% A (0.1% TFA-water):B (acetonitrile) to 100% A:B [0-5 minute: 5% A; 5-45 minute linear gradient to 100% B 2.375%/minute gradient] with a 20 mL/minute flowrate. Fractions containing the title compound were combined and lyophilized to give the title compound as a trifluoroacetate salt. ¹H NMR (600 MHz, DMSO-*d*₆) *δ* ppm 12.61 (s, 1H), 8.51 (dd, *J* = 2.4, 0.7 Hz, 1H), 8.27 (dd, *J* = 7.8, 1.4 Hz, 1H), 7.97 (d, *J* = 8.0 Hz, 1H), 7.92 - 7.80 (m, 4H), 7.44 (d, *J* = 7.8 Hz, 1H), 7.39 (t, *J =* 7.6 Hz, 3H), 7.28 (s, 2H), 6.90 (d, *J =* 9.2 Hz, 1H), 4.80 (s, 1H), 4.50 (s, 1H), 4.36 (s, 3H), 4.31 (s, 1H), 4.19 (s, 2H), 3.74 (s, 1H), 3.56 - 3.51 (m, 3H), 3.37 - 3.32 (m, 2H), 3.26 (s, 1H), 3.16 (s, 4H). ESI-MS *m*/*z* 637.81 (M+H)⁺.

### Probe 1G

### 6-[2-({2-[2-(2-aminoethoxy)ethoxy]ethoxy}methyl)-4-{3-[(4-oxo-3,4-dihydrophthalazin-1-yl)methyl]benzoyl}piperazin-1-yl]pyridine-3-carbonitrile

To 6-[2-({2-[2-(2-azidoethoxy)ethoxy]ethoxy}methyl)-4-({3-[(4-oxo-3,4-dihydrophthalazin-1-yl)methyl]phenyl}methyl)piperazin-1-yl]pyridine-3-carbonitrile trifluoroacetate salt (31 mg, 0.049 mmol) in tetrahydrofuran was added palladium on carbon (25.9 mg, 0.024 mmol) and the reaction mixture was stirred under H₂ at 1 atm for 16 hours. The reaction mixture was filtered and the filtrate was purified on a silica gel cartridge, eluting with NH₄OH/CH₃OH/CH₂Cl₂ to provide the title compound Analytical LCMS TFA method [gradient of 5-100% acetonitrile (A) and 0.1% trifluoroacetic acid in water (B) was used, at a flow rate of 1.5 mL/minute (0-0.05 minute 5% A, 0.05-1.2 minute 5-100% A, 1.2-1.4 minute 100% A, 1.4-1.5 min 100-5% A. 0.25 min post-run delay]: Rt = 0.73 min, ESI-MS *m*/*z* 612.5 (M+H)⁺.

### Probe 1H

### N-{2-[2-(2-{[1-(5-cyanopyridin-2-yl)-4-{3-[(4-oxo-3,4-dihydrophthalazin-1-yl)methyl]benzoyl}piperazin-2-yl]methoxy}ethoxy)ethoxy]ethyl }-2', 7'-difluoro-3', 6'-dihydroxy-3-oxo-3H-spiro[[2]benzofuran-1,9'-xanthene]-5-carboxamide

In Probe 1H, 6-[2-({2-[2-(2-Aminoethoxy)ethoxy]ethoxy}methyl)-4-{3-[(4-oxo-3,4-dihydrophthalazin-1-yl)methyl]benzoyl}piperazin-1-yl]pyridine-3-carbonitrile and 2,5-dioxopyrrolidin-1-yl 2',7'-difluoro-3',6'-dihydroxy-3 -oxo-3*H*-spiro[isobenzofuran-1,9'-xanthene]-5-carboxylate (8.79 mg, 0.017 mmol) were combined in anhydrous *N,N*-dimethylformamide containing 2% *N*,*N-*diisopropylethylamine (v/v) and the mixture was shaken at ambient temperature overnight. The reaction mixture was diluted to 3 mL with 90% dimethyl sulfoxide/water and was purified in one injection on a Gilson^{®} RP-HPLC running uniPoint software with a Waters^{™} Deltapak C18 200 x 25 mm column (15 um particle size, 100 Angstrom porosity) and was eluted with a gradient of 5% A (0.1% TFA-water):B (acetonitrile) to 100% A:B [0-5 minutes: 5% A; 5-45 minutes linear gradient to 100% B 2.375%/minutes gradient] with a 20 mL/minute flowrate. Fractions containing the title compound were combined and lyophilized to give the title compound as a trifluoroacetate salt. Analytical LCMS FA method [A gradient of 5-100% acetonitrile (A) and 0.1% formic acid water: acetonitrile (98:2) (B) was used, at a flow rate of 1.5 mL/minute (0-0.05 minute 0% A, 0.05-2.8 minute 0-100% A, 2.8-3.0 minute 100-0% A, 160-1500 amu positive/negative ESI-MS ionization]: Rt = 1.493 minute, ESI-MS *m*/*z* 503.8 (M+2H)²⁺, 1006.4 (M+H)⁺, 1005.0 (M-H)⁻; analytical LCMS AA method [A gradient of 5-100% acetonitrile (A) and 10 mM ammonium acetate water: acetonitrile (98:2) (B) was used, at a flow rate of 1.5 mL/minute (0-0.05 minute 0% A, 0.05-2.8 minute 0-100% A, 2.8-3.0 minute 100-0% A, 160-1500 amu positive/negative ESI-MS ionization]: Rₜ = 1.016 minute, ESI-MS *m*/*z* 503.4 (M+2H)²⁺, 1028.0 (M+Na)⁺, 1003.8 (M-H)⁻.

### SYNTHESIS OF PROBE 2

### Probe 2

### 2',7'-difluoro-N-[2-(2-{2-[{[4-(8-fluoro-6-oxo-3,4,5,6-tetrahydro-1H-azepino[5,4,3-cd]indol-2-yl)phenyl]methyl}(methyl)amino]ethoxy}ethoxy)ethyl]-3',6'-dihydroxy-3-oxo-3H-spiro[[2]benzofuran-1,9'-xanthene]-5-carboxamide

### Probe 2A

### tert-butyl [2-(2-{2-[{[4-(8-fluoro-6-oxo-3,4,5,6-tetrahydro-1H-azepino[5,4,3-cd]indol-2-yl)phenyl]methyl}(methyl)amino]ethoxy}ethoxy)ethyl]carbamate

In Probe 2A, 8-Fluoro-2-{4-[(methylamino)methyl]phenyl}-1,3,4,5-tetrahydro-6*H-*azepino[5,4,3-*cd*]indol-6-one hydrochloride (100.45 mg, 0.253 mmol) was combined with 2,2-dimethyl-4-oxo-3,8,11-trioxa-5-azatridecan-13-yl methanesulfonate (Julie Moreau and Jacqueline Marchand-Brynaert, Eur. J. Org. Chem.,2011, 1641-1644; 124 mg, 0.380 mmol) in 1 mL anhydrous *N,N*-dimethylformamide. Potassium carbonate (105 mg, 0.760 mmol) was added, and the reaction mixture was stirred at 70 °C for 16 hours. The reaction mixture was diluted with 25 mL saturated aqueous NH₄Cl and was extracted with 3 x 15 mL dichloromethane. The combined organic layers were dried over MgSO₄, filtered, and concentrated. The residue was diluted to 4 mL using 90% DMSO/water and was purified in 2 injections on a Gilson^{®} RP-HPLC running uniPoint software with a Waters^{™} Deltap C18 200 x 25 mm column (15 um particle size, 100 Angstrom porosity) and was eluted with a gradient of 5% A (0.1% TFA-water):B (acetonitrile) to 100% A:B [0-5 minutes: 5% A; 5-45 minute linear gradient to 100% B 2.375%/minute gradient] with a 20 mL/minute flowrate. Fractions containing the title compound were combined and lyophilized to give the title compound as the trifluoroacetate salt. ¹H NMR (600 MHz, DMSO-*d*₆) *δ* ppm 11.77 (s, 1H), 9.71 (s, 1H), 8.28 (t, *J =* 5.8 Hz, 1H), 7.76 - 7.72 (m, 2H), 7.69 - 7.64 (m, 2H), 7.45 (dd, *J* = 10.9, 2.4 Hz, 1H), 7.36 (dd, *J* = 9.1, 2.5 Hz, 1H), 6.76 (t, *J* = 5.7 Hz, 1H), 4.52 - 4.45 (m, 1H), 4.34 (dd, *J* = 12.8, 5.3 Hz, 1H), 3.80 (t, *J* = 5.3 Hz, 2H), 3.63 - 3.53 (m, 8H), 3.40 (q, *J =* 5.0 Hz, 4H), 3.34 (s, 1H), 3.28 - 3.22 (m, 1H), 3.07 *(q, J* = 6.0 Hz, 4H), 2.78 (d, *J =* 4.4 Hz, 3H), 1.37 (d, *J =* 1.9 Hz, 1H), 1.35 (s, 8H). ESI-MS *m*/*z* 555.51 (M+H)⁺.

### Probe 2B

### 2-(4-{[{2-[2-(2-aminoethoxy)ethoxy]ethyl}(methyl)amino]methyl}phenyl)-8-fluoro-1,3,4,5-tetrahydro-6H-azepino[5,4,3-cd]indol-6-one

In Probe 2B, *tert*-Butyl [2-(2-{2-[{[4-(8-fluoro-6-oxo-3,4,5,6-tetrahydro-1*H-*azepino[5,4,3-*cd*]indol-2-yl)phenyl]methyl}(methyl)amino]ethoxy}ethoxy)ethyl]carbamate trifluoroacetate salt (21.63 mg, 0.032 mmol) was dissolved in trifluoroacetic acid (1 mL) and immediately evaporated to dryness to give the title compound as the trifluoroacetate salt. ESI-MS *m*/*z* 455.04 (M+H)⁺.

### Probe 2C

### 2',7'-difluoro-N-[2-(2-{2-[{[4-(8-fluoro-6-oxo-3,4,5,6-tetrahydro-1H-azepino[5,4,3-cd]indol-2-yl)phenyl]methyl}(methyl)amino]ethoxy}ethoxy)ethyl]-3',6'-dihydroxy-3-oxo-3H-spiro[[2]benzofuran-1,9'-xanthene]-5-carboxamide

In Probe 2C, 2-(4- {[{2-[2-(2-aminoethoxy)ethoxy]ethyl}(methyl)amino]methyl}phenyl)-8-fluoro-1,3,4,5-tetrahydro-6*H*-azepino[5,4,3-cd]indol-6-one bis trifluoroacetate salt (22 mg, 0.032 mmol) and 2,5-dioxopyrrolidin-1-yl 2',7'-difluoro-3',6'-dihydroxy-3-oxo-3*H-*spiro[[2]benzofuran-1,9'-xanthene]-5-carboxylate (ThermoFisher Scientific, 18.06 mg, 0.035 mmol) were combined in 1 mL anhydrous dimethylformamide containing 2% diisopropylethylamine (v/v) and shaken at ambient temperature for 24 hours. The reaction mixture was diluted to 3 mL with 90% dimethyl sulfoxide/water and was purified in 1 injection with time collection on a Phenomenex^{®} Gemini^{®} 5 µM NX-C18 110 Angstrom 250 x 21.2 mm column and was eluted with a gradient A (0.1% TFA-water):B (CH₃CN) [0-5 minutes: 5% A; 5-35 minute linear gradient to 100% B 3.16%/minutes gradient] with a 20 mL/minute flowrate. Fractions containing the title compound were combined and lyophilized to give the title compound as a trifluoroacetate salt. Analytical LCMS TFA method [gradient of 5-100% acetonitrile (A) and 0.1% trifluoroacetic acid in water (B) was used, at a flow rate of 1.5 mL/minute (0-0.05 minute 5% A, 0.05-1.2 minutes 5-100% A, 1.2-1.4 minutes 100% A, 1.4-1.5 minutes 100-5% A. 0.25 minute post-run delay]: Rt = 0.68 minute, ESI-MS *m*/*z* 425.4 (M+2H)²⁺, 849.6 (M+H)⁺.

### TIME RESOLVED-FLUORESCENCE RESONANCE ENERGY TRANSFER (TR-FRET) ASSAY

TR-FRET assays were used to measure the binding of compounds to TIPARP and PARP1, and to characterize biochemical selectivity. Compound stock solutions (10 mM) were serially diluted in 3-fold increments in dimethyl sulfoxide (DMSO). Diluted compounds were dispensed (30 nL) into white ProxiPlate-384-well plates (PerkinElmer, Waltham, MA, USA) using an ECHO^{®} 550 acoustic Labcyte dispenser (Beckman-Coulter Life Sciences, Indianapolis, IN, USA) to achieve a final starting concentration of 30 µM to 0.5 nM in the assay. This was followed by addition of 5 µL of 2X protein/probe mix (Table 2) made in assay buffer (Tris-HCl 50 mM pH 8.0; 5 mM MgCl₂; 5 µM ZnCl₂; 1 mM DTT (dithiothreitol) ; 0.15% BSA (bovine serum albumin) (w/v); and 0.01% Triton X-100 (w/v)) at final concentrations listed in Table 2. Subsequently, 5 µL of 2X antibody (Tb-labeled anti-GST antibody, Thermo Fisher Scientific, Waltham, MA, USA) in HEPES buffered saline was added to each well at a final concentration of 1 nM. The samples were then incubated for 2 hours under ambient conditions. Fluorescence was measured and TR-FRET ratios were determined on an EnVision multimode plate reader (PerkinElmer) using a 520 nanometer (nm) excitation wavelength and collecting fluorescence emission at 495 nm. Results are shown in Table 3. As shown in Table 3, compounds in keeping with Formula (I) as described herein exhibited high potency for inhibiting TIPARP and comparatively lower inhibition of PARP1 (*i.e.,* high selectivity for TIPARP inhibition).

**Table 2**

| **Protein** | **Probe** | **Protein (nM)** | **Probe (nM**) |
|---|---|---|---|
| TIPARP GST-(TEV) | Probe 1 | 1 | 100 |
| PARP1 GST* | Probe 2 | 4 | 10 |

| | | | |
|---|---|---|---|
| *BPS BioScience Inc., San Diego, CA, USA | | | |

**Table 3**

| **Ex. #** | **TIPARP GST-(TEV) IC₅₀ (µM)** | **PARP1 GST IC₅₀ (µM)** | **PARP1/TIPARP IC₅₀ Ratio** | **Ex. #** | **TIPARP GST-(TEV) IC₅₀ (µM)** | **PARP1 GST IC₅₀ (µM)** | **PARP1/TIPARP IC₅₀ Ratio** |
|---|---|---|---|---|---|---|---|
| **1** | 0.00240 | 2.74 | 1140 | **16** | 0.00334 | 1.16 | 347 |
| **2** | 0.00375 | 5.02 | 1340 | **17** | 0.00500 | 0.206 | 41.2 |
| **3** | 0.00474 | 0.504 | 106 | **18** | 0.00586 | 0.871 | 149 |
| **4** | 0.00570 | 3.12 | 547 | **19** | 0.00579 | 6.75 | 1170 |
| **5** | 0.00230 | 2.24 | 974 | **20** | 0.00695 | 2.13 | 306 |
| **6** | 0.00343 | 0.283 | 82.5 | **21** | 0.0129 | 0.331 | 25.7 |
| **7** | 0.00379 | 1.58 | 417 | **22** | 0.00280 | 2.73 | 975 |
| **8** | 0.00879 | 1.20 | 137 | **23** | 0.00274 | 0.136 | 49.6 |
| **9** | 0.00432 | 4.36 | 1010 | **24** | 1.87 | 3.08 | 1.65 |
| **10** | 0.0101 | 3.08 | 305 | **25** | 2.00 | 2.03 | 1.02 |
| **11** | 0.0109 | 0.616 | 56.5 | **26** | 1.62 | 1.28 | 0.790 |
| **12** | 0.00316 | 3.65 | 1160 | **27** | 0.00234 | 0.231 | 98.7 |
| **13** | 0.00379 | 7.38 | 1950 | **28** | 0.00569 | 0.507 | 89.1 |
| **14** | 0.00217 | 4.11 | 1890 | **29** | 0.00175 | 0.263 | 150 |
| **15** | 0.00215 | 0.417 | 194 | | | | |

### CELLTITER-GLO^{®} CELL ASSAY FOR TIPARP INHIBITION

NCI-H1373 cells have been shown to undergo cell cycle arrest following inhibition of TIPARP. The NCI-H1373 CellTiter-Glo^{®} assay was used to determine cell numbers after TIPARP inhibition-induced cell cycle arrest, and enabled screening of compounds for cellular TIPARP inhibition potency.

Compound stock solutions (10 mM) were serially diluted in 3-fold increments in dimethyl sulfoxide. Sample aliquots (120 nL) were dispensed into Corning^{®} 384-well Flat Clear Bottom White Polystyrene TC-treated Microplates (Corning Life Sciences, Corning, NY, USA) using an ECHO^{®} 550 acoustic Labcyte dispenser (Beckman-Coulter Life Sciences, Indianapolis, IN, USA) to achieve final concentrations between 30 µM and 0.5 nM in the assay. This was followed by the addition of 40 µL of 1000 NCI-H1373 cells (ATCC^{®}, Manassas, VA, USA) in complete growth medium RPMI 1640 growth medium (Thermo Fisher Scientific, Waltham, MA, USA) supplemented with 20 mg/L L-glutamate, 25 mM HEPES (4-(2-hydroxyethyl)-1-piperazineethanesulfonic acid), 10% (v/v) heat inactivated FBS (fetal bovine serum), 1% (w/v) penicillin-streptomycin, and 100 nM 10-Cl-BBQ (10-chloro-7H-benzimidazo[2,1-a]benz[de]isoquinolin-7-one, (Tocris, Minneapolis, MN, USA). Duplicate cell plates with 40 µL cells/well along with various concentrations of compounds were kept in Nunc^{™} Square BioAssay Dishes (Thermo Fisher Scientific) at 98% relative humidity in a 5% CO₂ incubator at 37 °C for 6 days. On day 7, plates were moved to ambient lab conditions for 10 minutes until the temperature equilibrated. Following equilibration, CellTiter-Glo^{®} Luminescent Cell Viability Assay reagent (Promega^{™}, Madison, WI, USA) was added (15 µL) to each well of the plates. After a 20 minutes incubation, luminescence was detected using a ViewLux^{™} microplate imager (PerkinElmer, Waltham, MA, USA). Data analysis was performed using GraphPad Prism (Dotmatics, Boston, MA, USA). Results are shown in Table 4.

**Table 4**

| **Ex. #** | **NCI-H1373 CTG EC₅₀ (µM)** | **Ex. #** | **NCI-H1373 CTG EC₅₀ (µM)** | **Ex. #** | **NCI-H1373 CTG EC₅₀ (µM)** |
|---|---|---|---|---|---|
| **1** | 0.0172 | **11** | 0.337 | **21** | 0.160 |
| **2** | 0.0322 | **12** | 0.0292 | **22** | 0.0270 |
| **3** | 0.00965 | **13** | 0.0333 | **23** | 0.0442 |
| **4** | 0.0111 | **14** | 0.0109 | **24** | 6.49 |
| **5** | 0.0344 | **15** | 0.0148 | **25** | 10.2 |
| **6** | 0.0454 | **16** | 0.0191 | **26** | 9.10 |
| **7** | 0.0717 | **17** | 0.0341 | **27** | 0.0387 |
| **8** | 0.0559 | **18** | 0.0667 | **28** | 0.174 |
| **9** | 0.0677 | **19** | 0.0712 | **29** | 0.0182 |
| **10** | 0.284 | **20** | 0.0817 | | |

### CELLTITER-GLO^{®} DLD-1 BRCA2 (-/-) AND DLD-1 PARENT CELL ASSAY FOR PARP 1/2 INHIBITION

The DLD-1 BRCA2 (-/-) CellTiter-Glo^{®} assay measured the synthetic lethality of PARP1/2 inhibitors and was used as a surrogate to allow measurement of activity of both PARP1 and PARP2 in cells. If PARP1 and PARP2 are inhibited and are trapped onto chromatin during DNA replication, there is replication fork stalling and accumulation of double strand breaks. In BRCA wild-type cells, the resulting double strand DNA breaks are repaired via homologous recombination. In cells with mutation of BRCA1 or BRCA2, homologous recombination repair of DNA double strand breaks is defective, and cell death occurs after PARP1/2 inhibitor treatment (Lord CJ, Ashworth A. PARP inhibitors: synthetic lethality in the clinic. Science. 2017 March 17; 355(6330):1152-1158). Testing compounds in viability assays with the DLD-1-BRCA2 (-/-) and DLD-1 parent cell enabled assessment on PARP1/2-dependent (cytotoxicity in the BRCA2 (-/-) cells only) vs. non-selective cytotoxicity in both cell lines. Accordingly, viability assays with DLD-1 parent and DLD-1 BRCA2 cells demonstrated low PARP 1/2 inhibitory activity for compounds of Formula (I).

Compound stock solutions (10 mM) were serially diluted in 3-fold increments in dimethyl sulfoxide (DMSO). Sample aliquots (120 nL) were dispensed into Corning^{®} 384-well Flat Clear Bottom White Polystyrene TC-treated Microplates (Corning Life Sciences, Corning, NY, USA) using an ECHO^{®} 550 acoustic Labcyte dispenser (Beckman-Coulter Life Sciences, Indianapolis, IN, USA) to achieve concentrations between 30 µM and 0.5 nM in the assay. In the assay, 40 µL of 500 DLD-1 parent cells (HORIZON^{®} Therapeutics, Deerfield, IL, USA) or 40 µL of 1200 DLD-1-BRCA2 (-/-) cells (HORIZON^{®} Therapeutics) in complete growth medium containing RPMI 1640 growth medium (ThermoFisher Scientific, Waltham, MA, USA) supplemented with 20 mg/L L-glutamate, 25 mM HEPES (4-(2-hydroxyethyl)-1-piperazineethanesulfonic acid), 10% (v/v) heat inactivated FBS (fetal bovine serum), and 1% (w/v) Penicillin-Streptomycin were added.

Duplicate cell plates with 40 µL cells/well along with various concentrations of compounds were kept in Nunc^{™} Square BioAssay Dishes (Thermo Fisher Scientific) at 98% relative humidity in a 5% CO₂ incubator at 37 °C for 6 days. On day 7, plates were moved to ambient lab conditions for 10 minutes until the temperature equilibrated. Following equilibration, CellTiter-Glo^{®} Luminescent Cell Viability Assay reagent (Promega^{™}, Madison, WI, USA) was added (15 µL) to each well of the plates. After a 20 minutes incubation, luminescence was detected using a ViewLux^{™} microplate imager (PerkinElmer, Waltham, MA, USA). Data analysis was performed using GraphPad Prism (Dotmatics, Boston, MA, USA). Results are shown in Table 5. As shown in Table 5, DLD-1 parent and DLD-1 BRCA2 (-/-) cells showed good viability after incubation with compounds in keeping with Formula (I) as described herein (*i.e.,* low inhibition of PARP1/2).

**Table 5**

| **Ex. #** | **DLD-1 CTG EC₅₀ (µM)** | **DLD-1 BRCA2 (-/-) CTG EC₅₀ (µM)** | **Ex. #** | **DLD-1 CTG EC₅₀ (µM)** | **DLD-1 BRCA2 (-/-) CTG EC₅₀ (µM)** | **Ex. #** | **DLD-1 CTG EC₅₀ (µM)** | **DLD-1 BRCA2** (-/-) **CTG EC₅₀ (µM)** |
|---|---|---|---|---|---|---|---|---|
| **1** | >30 | 18.5 | **11** | 16.0 | 1.18 | **21** | >30 | 1.06 |
| **2** | 22.1 | 16.5 | **12** | 11.1 | 5.48 | **22** | >30 | 9.19 |
| **3** | >30 | 5.03 | **13** | 11.1 | 6.96 | **23** | 13.3 | 0.785 |
| **4** | 23.9 | 7.17 | **14** | 14.3 | 4.49 | **24** | >30 | 23.4 |
| **5** | >30 | 20.5 | **15** | 10.3 | 1.38 | **25** | >30 | >30 |
| **6** | 14.4 | 1.13 | **16** | 15.2 | 0.997 | **26** | >30 | >30 |
| **7** | >30 | 17.2 | **17** | 9.75 | 0.217 | **27** | >30 | 0.894 |
| **8** | >30 | 5.73 | **18** | 12.0 | 1.05 | **28** | >30 | 5.45 |
| **9** | >30 | >30 | **19** | >30 | 18.3 | **29** | 5.96 | 0.23 |
| **10** | >30 | >30 | **20** | >30 | 3.00 | | | |

### MC-38 (KER) SYNGENEIC MODEL EFFICACY

The murine cell line MC-38 derived from C57BL6 murine colon adenocarcinoma cells was obtained from Kerafast (Boston, MA, USA). A suspension of 2.5 x 10⁵ cells in culture medium mixed with Matrigel^{®} (Corning Life Sciences, Lowell, MA, USA; 1: 1, volume:volume) was injected subcutaneously in the right hind flank of female C57BL/6 mice to generate tumor formation. Treatment started when the size of the flank tumors was approximately 120 mm³. Vehicle and four different doses of Example 1 were administered once daily to each of eight mice at each dose level for up to 21 days. Each administration consisted of a single 0.2 mL oral (PO) dose in a vehicle formulation (10% ethanol, 30% PEG300, and 60% PHOSAL^{®} 50 PG).

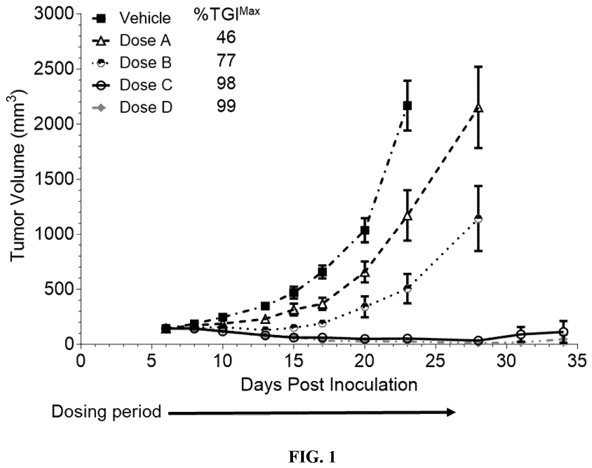

Results are depicted in FIG. 1. As shown, the administration of Example 1 inhibited the growth of MC-38 (Ker) tumors compared to vehicle. The maximum tumor growth inhibition (TGI^{Max}) achieved by Doses A-D were 46%, 77%, 98%, and 99%, respectively.

### TUMOR SELECTIVE INDUCTION OF IFNβ IN MC-38 (KER) SYNGENEIC MODEL

The murine cell line MC-38 derived from C57BL/6 murine colon adenocarcinoma cells was obtained from Kerafast (Boston, MA, USA). A suspension of 2.5 x 10⁵ cells in culture medium mixed with Matrigel^{®} (Corning Life Sciences, Lowell, MA, USA; 1: 1, volume:volume) was injected subcutaneously in the right hind flank of female C57BL/6 mice to generate tumor formation. Treatment started when the size of the flank tumors was approximately 500 mm³. Vehicle and three different doses of Example 1 were administered once daily to each of four to five mice at each dose level for 2 days. Each administration consisted of a single 0.2 mL PO dose in a vehicle formulation (10% ethanol, 30% PEG300, and 60% PHOSAL^{®} 50 PG).

Blood and tumors were collected 3 hours after the last dose. Blood was transferred to microfuge tubes containing EDTA, centrifuged at 2000 x g for 8 minutes at 4 °C, and the upper plasma layer was transferred to new tubes and frozen at -80 °C. Tumors were weighed and 200 to 500 mg of tumor tissue was transferred to microfuge tubes, which were then centrifuged at 15000 x g for 10 minutes at 4 °C. The supernatant was transferred to a fresh 96 well plate. 20 µL of cold Dulbecco's Phosphate Buffered Saline (DPBS) was added to the tubes containing the tumors and the tumors were dislodged with pipette tips. The tubes were then centrifuged at 500 x g for 10 minutes at 4 °C, and the supernatant was combined with the corresponding supernatant from the previous step in the 96 well plate. The plate containing the combined supernatants (tumor fluid) was centrifuged at 500 x g for 3 minutes at 4 °C, and clean supernatant was transferred to a new 96 well plate which was then frozen at -80 °C. The concentration of IFNβ in the thawed plasma and tumor fluid was determined using a Milliplex MAP mouse IFNβ assay (EMD Millipore, Burlington, MA, USA).

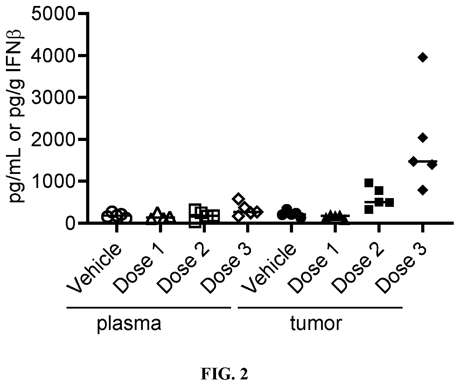

Results are depicted in FIG. 2. As shown, the administration of Example 1 induced IFNβ in MC-38 (Ker) tumors compared to vehicle but not in plasma, indicating a selective induction of cytokines in the tumor microenvironment.

### NCI-H1373 XENOGRAFT TUMOR MODEL EFFICACY

The human xenograft line NCI-H1373 was obtained from ATCC (American Type Culture Collection, Manassas, Virginia, USA). A suspension of 5 x 10⁶ cells in culture medium mixed with Matrigel^{®} (Corning Life Sciences, Lowell, MA, USA; 1: 1, volume:volume) was injected subcutaneously in the right hind flank of female SCID-beige mice to generate tumor formation. Treatment started when the size of the flank tumors was approximately 120 mm³. The TIPARP inhibition activity of Example 1 was evaluated by dosing five separate treatment groups with vehicle and four different doses of Example 1, administered twice a day (BID) for 14 days. Each administration consisted of a 0.2 mL PO dose in a vehicle formulation (10% ethanol, 30% PEG300, and 60% PHOSAL^{®} 50).

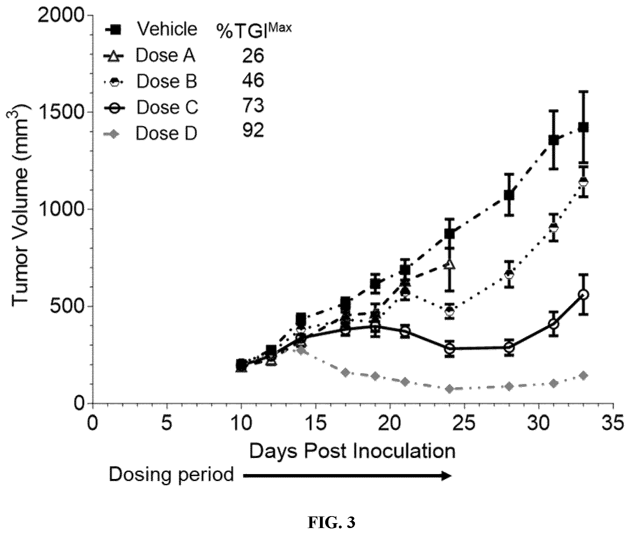

Results are depicted in FIG. 3. As shown, the administration of Example 1 inhibited the growth of NCI-H1373 tumors compared to vehicle. The TGI^{Max} achieved by Doses A-D were 26%, 46%, 73%, and 92%, respectively.

## Claims

1. A compound of Formula (I), or a pharmaceutically acceptable salt thereof, 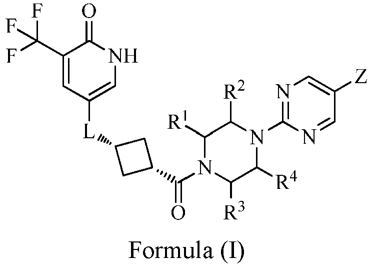 wherein:
L is selected from the group consisting of a bond and CH₂;
R¹, R², R³, and R⁴ are independently selected from the group consisting of H and CH₃, wherein at least one and no more than two of R¹, R², R³, and R⁴ are CH₃; and
Z is selected from the group consisting of CF₃ and C(CH₃)₂OH.

2. The compound of claim 1, or a pharmaceutically acceptable salt thereof, wherein L is a bond.

3. The compound of claim 2, or a pharmaceutically acceptable salt thereof, wherein one of R¹ or R⁴ is CH₃; both R¹ and R⁴ are CH₃; one of R² or R³ is CH₃; or both R² and R³ are CH₃.

4. The compound of claim 2 or 3, or a pharmaceutically acceptable salt thereof, wherein Z is CF₃.

5. The compound of claim 1, or a pharmaceutically acceptable salt thereof, wherein L is CH₂.

6. The compound of claim 5, or a pharmaceutically acceptable salt thereof, wherein one of R¹ or R⁴ is CH₃; both R¹ and R⁴ are CH₃; one of R² or R³ is CH₃; or both R² and R³ are CH₃.

7. The compound of claim 5 or 6, or a pharmaceutically acceptable salt thereof, wherein Z is C(CH₃)₂OH.

8. The compound of claim 1, or a pharmaceutically acceptable salt thereof, wherein the compound is selected from the group consisting of:
5-[(1*S*,3*s*)-3-{(2*R*)-2-methyl-4-[5-(trifluoromethyl)pyrimidin-2-yl]piperazine-1-carbonyl }cyclobutyl]-3-(trifluoromethyl)pyridin-2(14)-one;
5-[{1*s*,3*s*)-3-{(2*R*,6*S*)-2,6-dimethyl-4-[5-(tiifluoromethyl)pyrimidin-2-yl]piperazine-1-carbonyl }cyclobutyl]-3-(trifluoromethyl)pyridin-2(14)-one;
5-[(1*S*,3*s*)-3-{(3*R*)-3-methyl-4-[5-(trifluoromethyl)pyrimidin-2-yl]piperazine-1-carbonyl }cyclobutyl]-3-(trifluoromethyl)pyridin-2(14)-one;
5-[(1*s*,3*s*)-3-f (3*R*,5*S*)-3,5-dimethyl-4-[5-(tiifluoromethyl)pyrimidin-2-yl]piperazine-1-carbonyl}cyclobutyl]-3-(trifluoromethyl)pyridin-2(14)-one;
5-[(1*s*,3*s*)-3-{(3*R*,5*S*)-4-[5-(2-hydroxypropan-2-yl)pyrimidin-2-yl]-3,5-dimethylpiperazine-1-carbonyl}cyclobutyl]-3-(trifluoromethyl)pyridin-2(1*H*)-one;
5-[(1*R*,3*s*)-3-{(2*S*)-2-methyl-4-[5-(trifluoromethyl)pyrimidin-2-yl]piperazine-1-carbonyl }cyclobutyl]-3-(trifluoromethyl)pyridin-2(14)-one;
5-[(1*S*,3*s*)-3-{(3*R*)-4-[5-(2-hydroxypropan-2-yl)pyrimidin-2-yl]-3-methylpiperazine-1-carbonyl}cyclobutyl]-3-(trifluoromethyl)pyridin-2(1*H*)-one;
5-[(1*R*,3*s*)-3-{(3*S*)-3-methyl-4-[5-(trifluoromethyl)pyrimidin-2-yl]piperazine-1-carbonyl}cyclobutyl]-3-(trifluoromethyl)pyridin-2(1*H*)-one;
5-[(1*S*,3*s*)-3-{(2*R*)-4-[5-(2-hydroxypropan-2-yl)pyrimidin-2-yl]-2-methylpiperazine-1-carbonyl}cyclobutyl]-3-(trifluoromethyl)pyridin-2(1*H*)-one;
5-[(1*R*,3*s*)-3-{(3*S*)-4-[5-(2-hydroxypropan-2-yl)pyrimidin-2-yl]-3-methylpiperazine-1-carbonyl}cyclobutyl]-3-(trifluoromethyl)pyridin-2(1*H*)-one;
5-[(1*R*,3*s*)-3-{(2*S*)-4-[5-(2-hydroxypropan-2-yl)pyrimidin-2-yl]-2-methylpiperazine-1-carbonyl } cyclobutyl]-3-(trifluoromethyl)pyridin-2(14)-one;
5-{[(1*S*,3*s*)-3-{(2*R*)-2-methyl-4-[5-(trifluoromethyl)pyrimidin-2-yl]piperazine-1-carbonyl}cyclobutyl]methyl}-3-(trifluoromethyl)pyridin-2(1*H*)-one;
5-{[(1*s*,3*s*)-3-{(2*R*,6*S*)-2,6-dimethyl-4-[5-(trifluoromethyl)pyrimidin-2-yl]piperazine-1-carbonyl}cyclobutyl]methyl}-3-(trifluoromethyl)pyridin-2(1*H*)-one;
5-{[(1*s*,3*s*)-3-{(3*R*,5*S*)-3,5-dimethyl-4-[5-(trifluoromethyl)pyrimidin-2-yl]piperazine-1-carbonyl}cyclobutyl]methyl}-3-(trifluoromethyl)pyridin-2(1*H*)-one;
5-{[(1*S*,3*s*)-3-{(3*R*)-3-methyl-4-[5-(trifluoromethyl)pyrimidin-2-yl]piperazine-1-carbonyl}cyclobutyl]methyl}-3-(trifluoromethyl)pyridin-2(1*H*)-one;
5-{[(1*R*,3*s*)-3-{(3*S*)-3-methyl-4-[5-(trifluoromethyl)pyrimidin-2-yl]piperazine-1-carbonyl}cyclobutyl]methyl}-3-(trifluoromethyl)pyridin-2(1*H*)-one;
5-{[(1*R*,3*s*)-3-{(2*S*)-2-methyl-4-[5-(trifluoromethyl)pyrimidin-2-yl]piperazine-1-carbonyl}cyclobutyl]methyl}-3-(trifluoromethyl)pyridin-2(1*H*)-one;
5-{[(1*S*,3*s*)-3-{(3*R*)-4-[5-(2-hydroxypropan-2-yl)pyrimidin-2-yl]-3-methylpiperazine-1-carbonyl}cyclobutyl]methyl}-3-(trifluoromethyl)pyridin-2(1*H*)-one;
5-{[(1*S*,3*s*)-3-{(2*R*)-4-[5-(2-hydroxypropan-2-yl)pyrimidin-2-yl]-2-methylpiperazine-1-carbonyl}cyclobutyl]methyl}-3-(trifluoromethyl)pyridin-2(1*H*)-one;
5-{[(1*R*,3*s*)-3-{(3*S*)-4-[5-(2-hydroxypropan-2-yl)pyrimidin-2-yl]-3-methylpiperazine-1-carbonyl}cyclobutyl]methyl}-3-(trifluoromethyl)pyridin-2(1*H*)-one;
5-{[(1*R*,3*s*)-3-{(2*S*)-4-[5-(2-hydroxypropan-2-yl)pyrimidin-2-yl]-2-methylpiperazine-1-carbonyl }cyclobutyl]methyl }-3-(trifluoromethyl)pyridin-2(1H)-one; and
5-{[(1*s*,3*s*)-3-{(3*R*,5*S*)-4-[5-(2-hydroxypropan-2-yl)pyrimidin-2-yl]-3,5-dimethylpiperazine-1-carbonyllcyclobutyl]methyl}-3-(tiifluoromethyl)pyiidin-2(1*H*)-one.

9. The compound of claim 8, wherein the compound is 5-[(1*S*,3*s*)-3-{(2*R*)-2-methyl-4-[5-(trifluoromethyl)pyrimidin-2-yl]piperazine-1-carbonyl}cyclobutyl]-3-(trifluoromethyl)pyridin-2(1H)-one, or a pharmaceutically acceptable salt thereof.

10. The compound of claim 9, wherein the compound is 5-[(1S,3s)-3-{(2R)-2-methyl-4-[5-(trifluoromethyl)pyrimidin-2-yl]piperazine-1-carbonyl}cyclobutyl]-3-(trifluoromethyl)pyridin-2(1H)-one.

11. The compound of claim 9, wherein the compound is a pharmaceutically acceptable salt of 5-[(1*S*,3*s*)-3-{(2*R*)-2-methyl-4-[5-(trifluoromethyl)pyrimidin-2-yl]piperazine-1-carbonyl}cyclobutyl]-3-(trifluoromethyl)pyridin-2(1*H*)-one.

12. A pharmaceutical composition comprising the compound of any previous claim, or a pharmaceutically acceptable salt thereof, and a pharmaceutically acceptable excipient.

13. A compound according to any one of claims 1 to 11 or a pharmaceutical composition according to claim 12, for use in a method for treating head and neck squamous cell carcinoma (HNSCC), the method comprising administering the compound, or a pharmaceutically acceptable salt thereof, or the pharmaceutical composition, to a human patient in need thereof.
